# EUROPEAN PATENT SPECIFICATION

(11) **EP 2 992 899 B1**
(45) Date of publication and mention of the grant of the patent: **01.07.2020**
(21) Application number: 15178476.6
(22) Date of filing: 07.05.2010
(51) Int. Cl.: A61K 39/39

(54) **ENHANCED IMMUNE RESPONSE IN AVIAN SPECIES**
VERBESSERTE IMMUNANTWORT BEI VOGELSPEZIES
AMPLIFICATION DE LA RÉACTION IMMUNITAIRE CHEZ L'ESPÈCE AVIAIRE

(30) Priority: 14.05.2009 US 178099 P
(43) Date of publication of application: 09.03.2016
(62) Divisional of application: 10720350.7
(73) Proprietor: Bayer Intellectual Property GmbH, 40789 Monheim am Rhein (DE)
(72) Inventor: Abraham, Albert, Shawnee, KS 66216 (US)
(74) Representative: Viering, Jentschura & Partner mbB Patent- und Rechtsanwälte

(56) References cited:
- WO-A2-99/66879
- Azita Taghavi: "Immunostimulatory effect of oligodeoxynucleotides containing CpG motifs (CpG-ODN) in neonatal broiler chickens", , 2008, XP002596617, Saskatoon Retrieved from the Internet: URL:http://library2.usask.ca/theses/availa ble/etd-04292008-141610/unrestricted/azita .pdf [retrieved on 2010-08-16]
- GURSEL I ET AL: "Sterically stabilized cationic liposomes improve the uptake and immunostimulatory activity of CpG oligonucleotides.", JOURNAL OF IMMUNOLOGY (BALTIMORE, MD. : 1950) 15 SEP 2001 LNKD- PUBMED:11544321, vol. 167, no. 6, 15 September 2001 (2001-09-15), pages 3324-3328, XP002596618, ISSN: 0022-1767
- SUAREZ D L ET AL: "The effect of eukaryotic expression vectors and adjuvants on DNA vaccines in chickens using an avian influenza model", AVIAN DISEASES, AMERICAN ASSOCIATION OF AVIAN PATHOLOGISTS, KENNET SQ., PA, US, vol. 44, no. 4, 1 October 2000 (2000-10-01), pages 861-868, XP009137123, ISSN: 0005-2086
- VERMINNEN KRISTEL ET AL: "Vaccination of turkeys against Chlamydophila psittaci through optimised DNA formulation and administration.", VACCINE 19 APR 2010 LNKD- PUBMED:20199760, vol. 28, no. 18, 19 April 2010 (2010-04-19), pages 3095-3105, XP002596619, ISSN: 1873-2518
- GOMIS SUSANTHA ET AL: "Protection of chickens against Escherichia coli infections by DNA containing CpG motifs.", INFECTION AND IMMUNITY, vol. 71, no. 2, February 2003 (2003-02), pages 857-863, XP002596620, ISSN: 0019-9567
- LÉVESQUE S ET AL: "Improvement of adjuvant systems to obtain a cost-effective production of high levels of specific IgY.", POULTRY SCIENCE APR 2007 LNKD- PUBMED:17369532, vol. 86, no. 4, April 2007 (2007-04), pages 630-635, XP002596621, ISSN: 0032-5791

## Description

### FIELD OF THE INVENTION

The present invention relates to a specific immunomodulator composition for use in preventing a reduction in survival rate when co-administered with a vaccine of a chicken hatched from an embryonated chicken egg, by administration *in ovo* to the egg of an effective amount of 0.05 to 10 micrograms of the immunomodulator composition, wherein the vaccine is a vaccine used for protection against Marek's disease virus (MDV).

### BACKGROUND OF THE INVENTION

Vaccines are used to prevent infectious disease and to treat established diseases. Infectious diseases are caused by infectious agents including such examples as viruses, bacteria, parasites, and other fungi. Many reagents and methods have been developed to prevent and treat infectious diseases for all types of species including mammals, birds, fish, and primates.

Vaccination programs are particularly important for commercially raised animals used in the food industry. Birds are prime targets for many types of infections. Breeders and commercial chicken, turkey, and other poultry flocks are routinely vaccinated to protect them against environmental exposure to pathogens. One of the most economically important diseases to the poultry industry is Marek's disease, which is a lymphoproliferative disease that occurs naturally in chickens. The disease is caused by a highly contagious herpesvirus that spreads horizontally, and has been responsible for major economic losses to the poultry industry. The symptoms of Marek's disease appear widely throughout the nerves, genital organs, internal organs, eyes and skin of infected birds causing motor paralysis, decreased organ functionality, and chronic undernourishment ultimately leading to death.

Hatching birds are exposed to pathogenic microorganisms shortly after birth. Although these birds are initially protected against pathogens by maternally derived antibodies, this protection is only temporary, and the bird's own immature immune system must begin to protect the bird against pathogens. It is often desirable to prevent infection in young birds when they are most susceptible. It is also desirable to prevent against infection in older birds, especially when the birds are housed in close-quarters, leading to the rapid spread of disease.

In most commercial flocks, newly hatched chicks are given certain vaccines parenterally at hatch. Because exposure to pathogens often occurs at a very young age, they often need to be vaccinated before they are placed in rearing or brooder houses. Such a vaccination scheme requires handling of individual birds and involves the possible risk of accidental self-injection. Further, these vaccines are not always effective. The young chicks may become exposed to a virulent form of a disease too soon after vaccination before they have the opportunity to develop adequate protective immunity.

Some live viral vaccines can be administered in eggs before the birds hatch. This procedure is referred to as "in ovo vaccination." The in ovo vaccinated birds develop resistance to the target disease. However, many vaccines used for hatched birds cannot be used for in ovo vaccination due to the embryonic pathogenicity of the vaccine agents. Late stage embryos are highly susceptible to infection with most vaccine viruses examined. Not all vaccine viruses that are non-pathogenic for newly hatched chicks are also safe for late-stage embryos. For instance, vaccine strains of infectious bronchitis virus (IBV) and Newcastle disease virus (NDV), that are used routinely as neonatal vaccines in newly hatched chicks, are lethal for embryos following in ovo inoculation. These viruses have been modified to render them safe for in ovo use. Modification of viruses weakens the immune response elicited and is therefore a less effective vaccine agent in protecting the late-stage embryos.

The vaccination program resulting from such disparate vaccines necessary to provide protection from infectious disease and economic loss is complex. Therefore a need exists for a method of eliciting a non-antigen-specific immune response that enhances protection of birds from infectious disease and is easy to administer. In addition, it is desirable to have a method of eliciting an immune response that provides a protective function for more than one infectious agent. Further a need exists for a method of eliciting an immune response that has a longer duration or that does not require booster shots of a vaccine. The use of CpG in a cationic liposome delivery vehicle for vaccinating *in ovo* and for protecting the hatched chicken against a challenge with E. coli has been described in the art (Taghavi et al. Avian Dis. 2008 Sep; 52(3):398-406). Additionally, it has been described in the art that treatment with CpG-ODN protects neonatal chickens against an intracellular bacterial infection and that co-treatment of CpG-ODN with polyphosphazene enhances the immunoprotective effect of CpG-ODN (Taghavi A. Immunostimulatory effect of oligodeoxynucleotides containing CpG motifs (CpG-ODN) in neonatal broiler chickens. Master thesis. Saskatoon. 2008). The present invention provides a specific immunomodulator composition for use in preventing a reduction in survival rate when co-administered with a vaccine of a chicken hatched from an embryonated chicken egg, as defined in the appended claims.

### BRIEF DESCRIPTION OF THE DRAWINGS

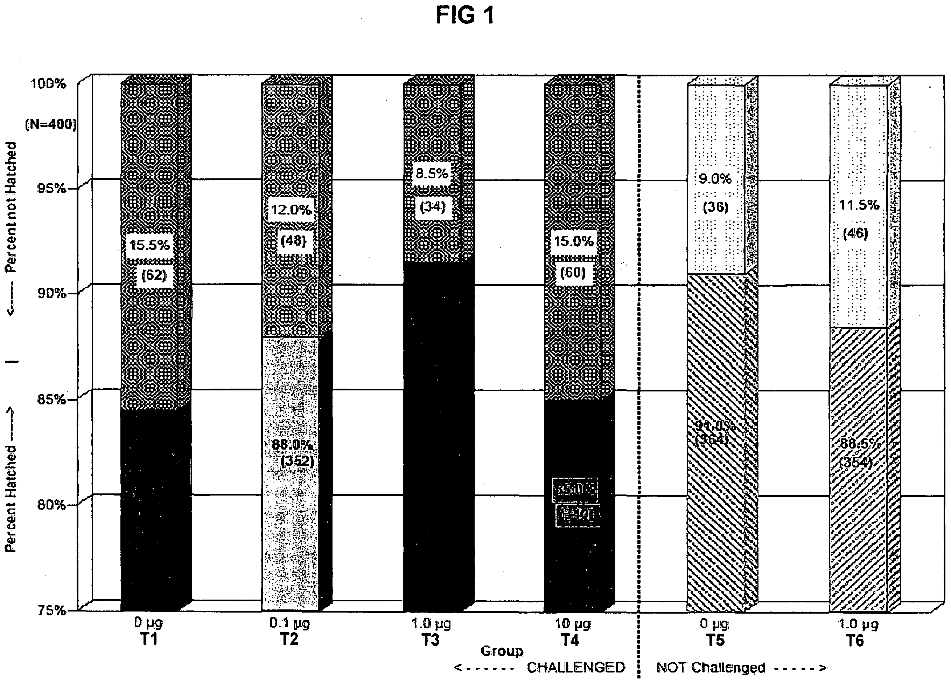

FIG. 1 graphically depicts the hatch rate between differentially treated groups of embryonated chicken eggs. The study groups analyzed include T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

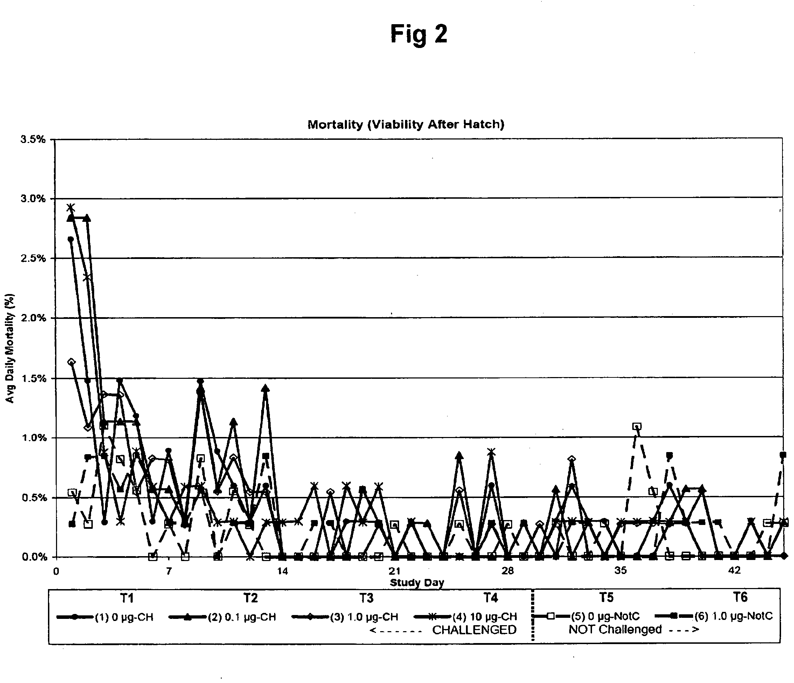

FIG. 2 graphically shows the average daily mortality per pen per day after hatch. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

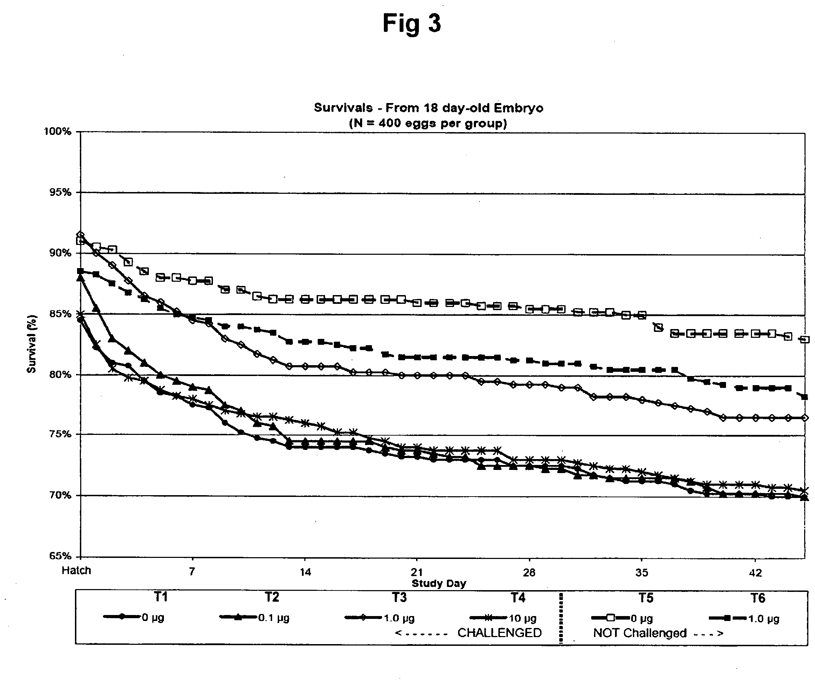

FIG. 3 graphically illustrates the proportion of birds surviving on any particular study day per pen based on the initial number of embryonated eggs. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

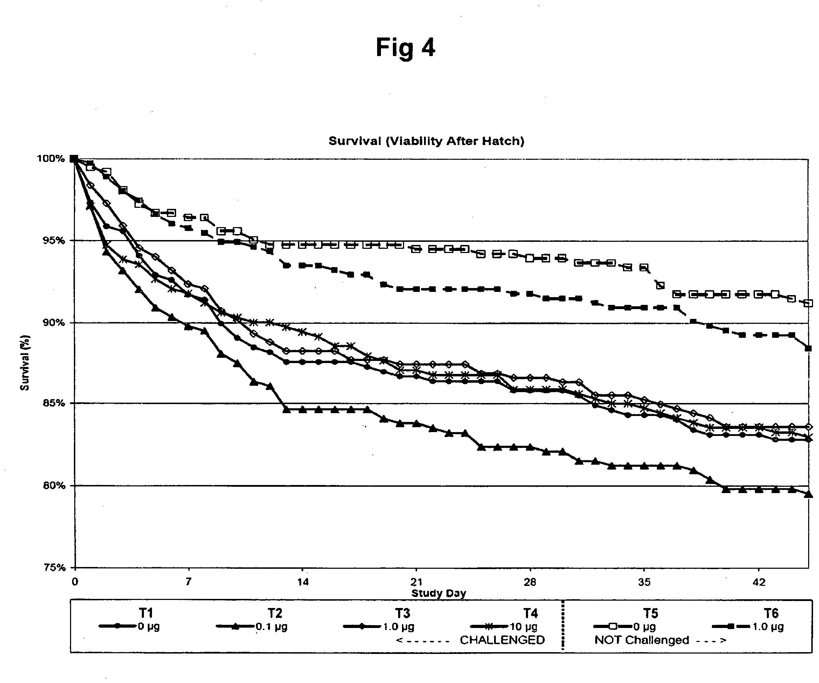

FIG. 4 graphically represents the viability of birds after hatch. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

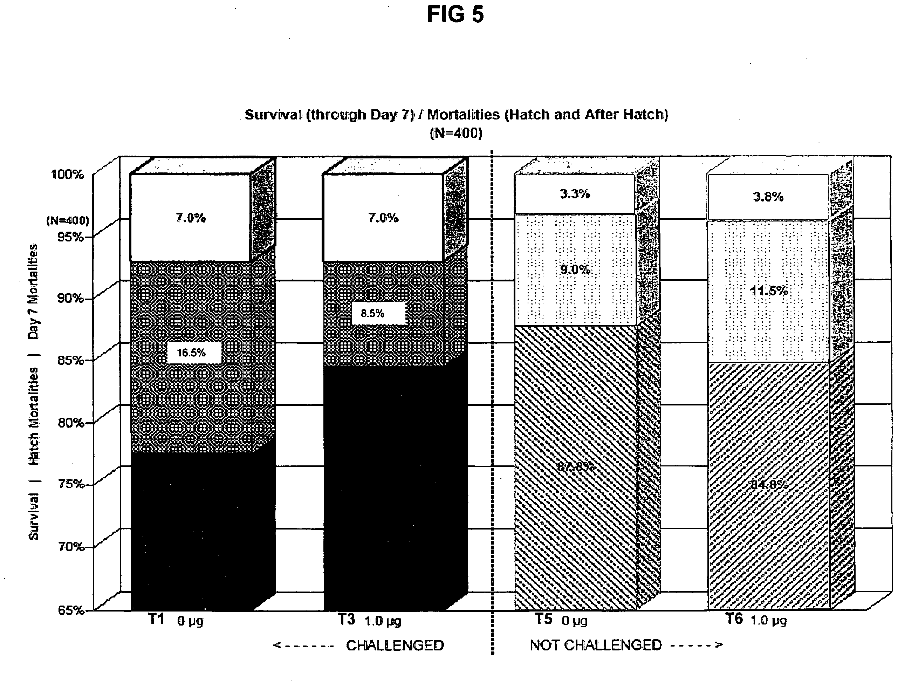

FIG. 5 graphically illustrates the survival rates, hatch mortalities, and after hatch mortalities through study day 7. Key: T1, 0 µg immunomodulator/egg and E. coli challenged; T3,1.0 µg immunomodulator/egg and E. coli challenged; T5, 0 µg immunomodulator/egg and not challenged; and T6, 1.0 µg immunomodulator/egg and not challenged.

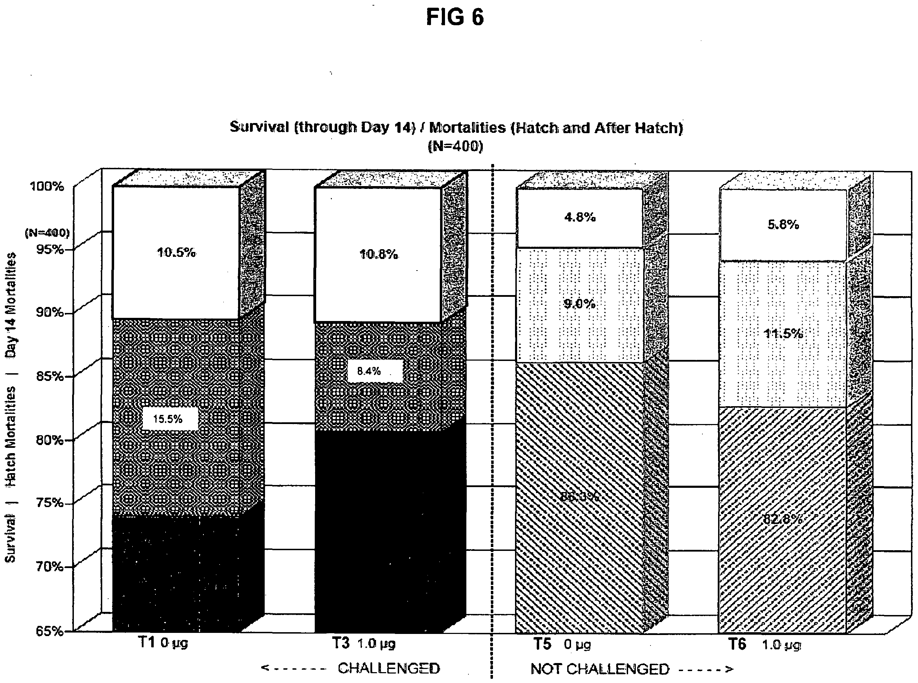

FIG. 6 graphically represents the survival rates, hatch mortalities, and after hatch mortalities through study day 14. Key: T1, 0 µg immunomodulator/egg and E. coli challenged; T3,1.0 µg immunomodulator/egg and E. coli challenged; T5, 0 µg immunomodulator/egg and not challenged; and T6, 1.0 µg immunomodulator/egg and not challenged.

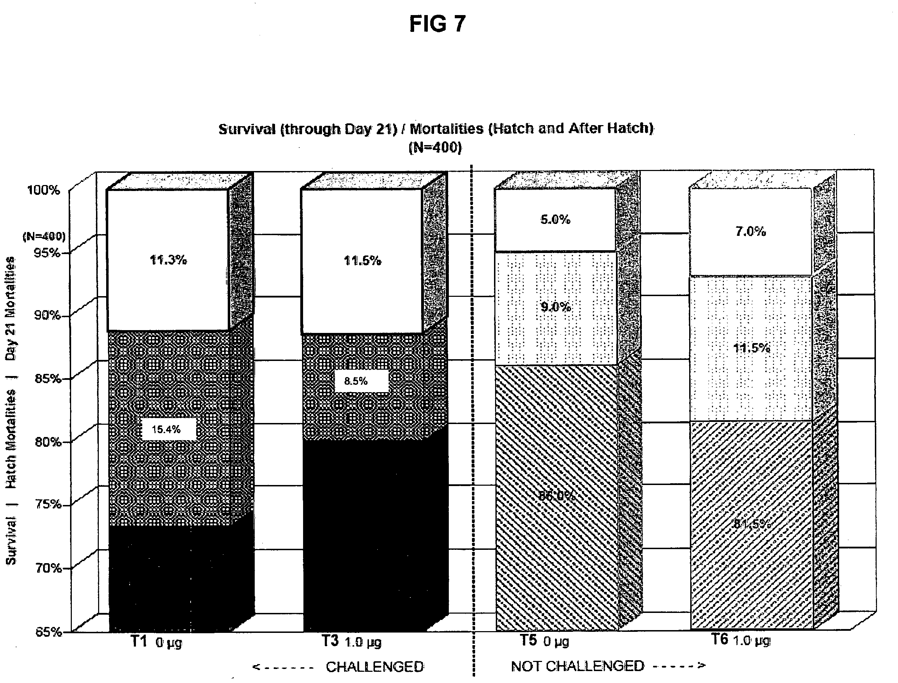

FIG. 7 graphically depicts the survival rates, hatch mortalities, and after hatch mortalities through study day 21. Key: T1, 0 µg immunomodulator/egg and E. coli challenged; T3,1.0 µg immunomodulator/egg and E. coli challenged; T5, 0 µg immunomodulator/egg and not challenged; and T6, 1.0 µg immunomodulator/egg and not challenged.

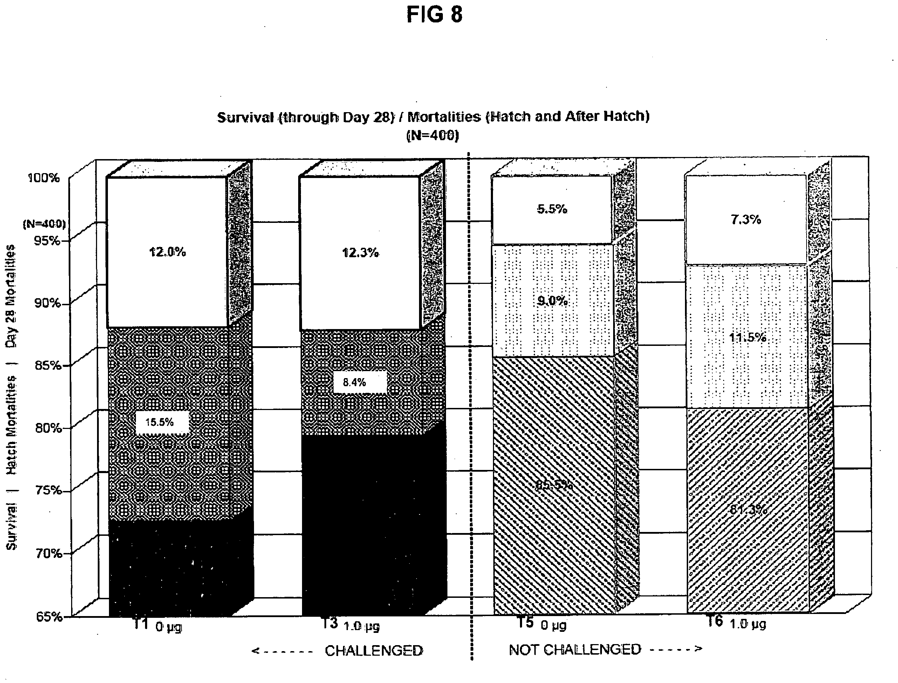

FIG. 8 graphically illustrates the survival rates, hatch mortalities, and after hatch mortalities through study day 28. Key: T1, 0 µg immunomodulator/egg and E. coli challenged; T3, 1.0 µg immunomodulator/egg and E. coli challenged; T5, 0 µg immunomodulator/egg and not challenged; and T6, 1.0 µg immunomodulator/egg and not challenged.

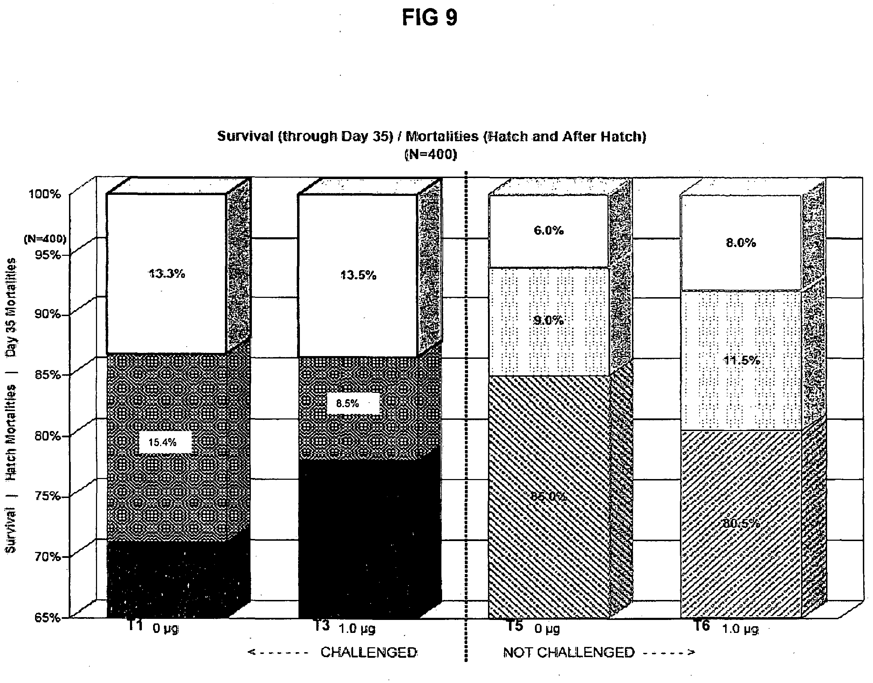

FIG. 9 graphically depicts the survival rates, hatch mortalities, and after hatch mortalities through study day 35. Key: T1, 0 µg immunomodulator/egg and E. coli challenged; T3, 1.0 µg immunomodulator/egg and E. coli challenged; T5, 0 µg immunomodulator/egg and not challenged; and T6, 1.0 µg immunomodulator/egg and not challenged.

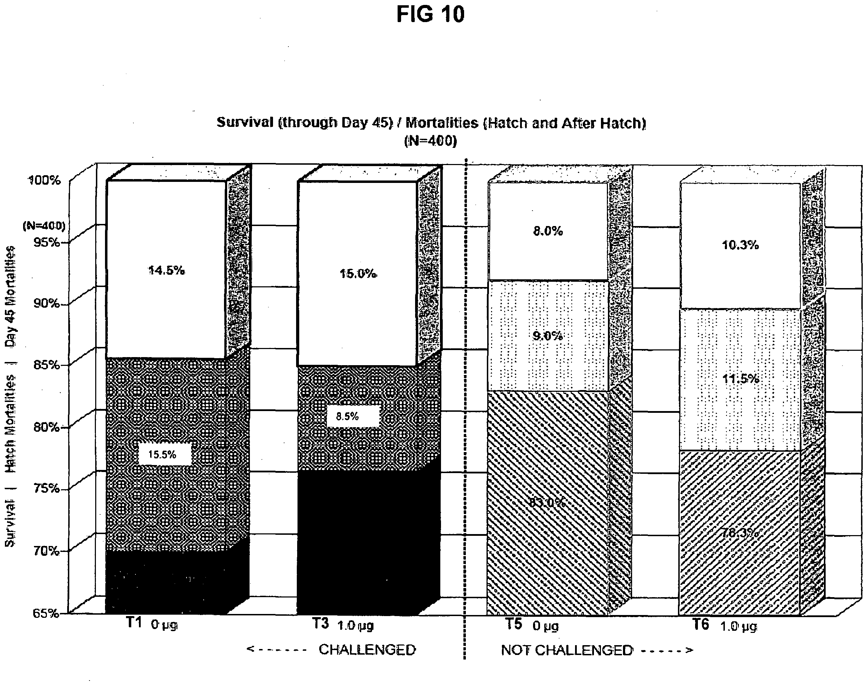

FIG. 10 graphically represents the survival rates, hatch mortalities, and after hatch mortalities through study day 45. Key: T1, 0 µg immunomodulator/egg and E. coli challenged; T3,1.0 µg immunomodulator/egg and E. coli challenged; T5, 0 µg immunomodulator/egg and not challenged; and T6, 1.0 µg immunomodulator/egg and not challenged.

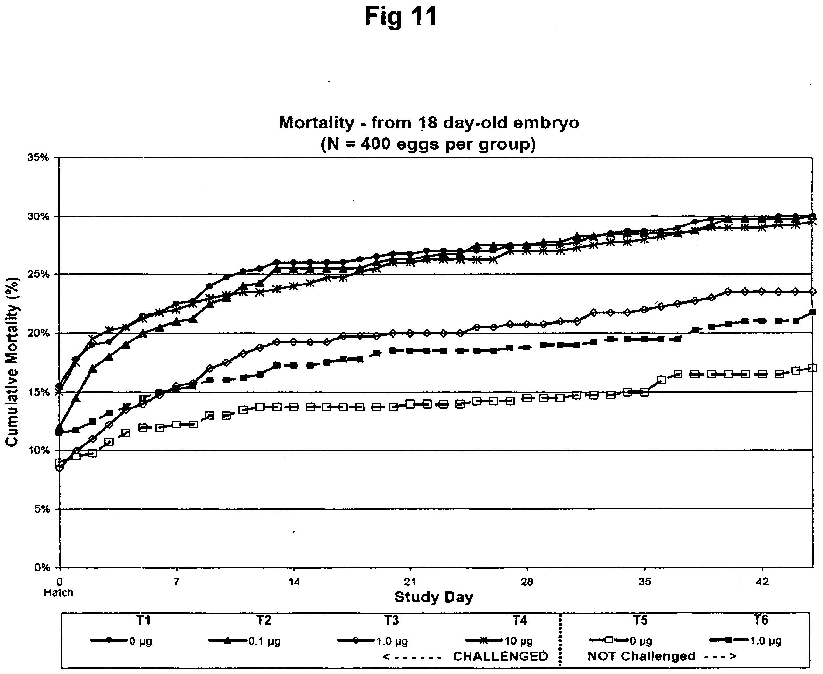

FIG. 11 shows the cumulative mortality rate per group from 18 day old embryos through study day 45. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

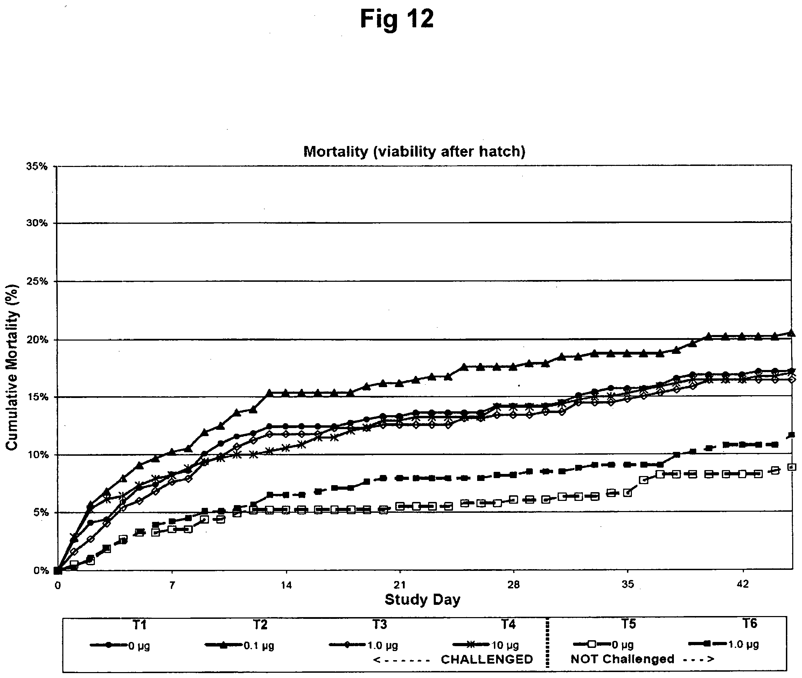

FIG. 12 shows the cumulative mortality rate per group after hatch, from study day 0 through study day 45. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

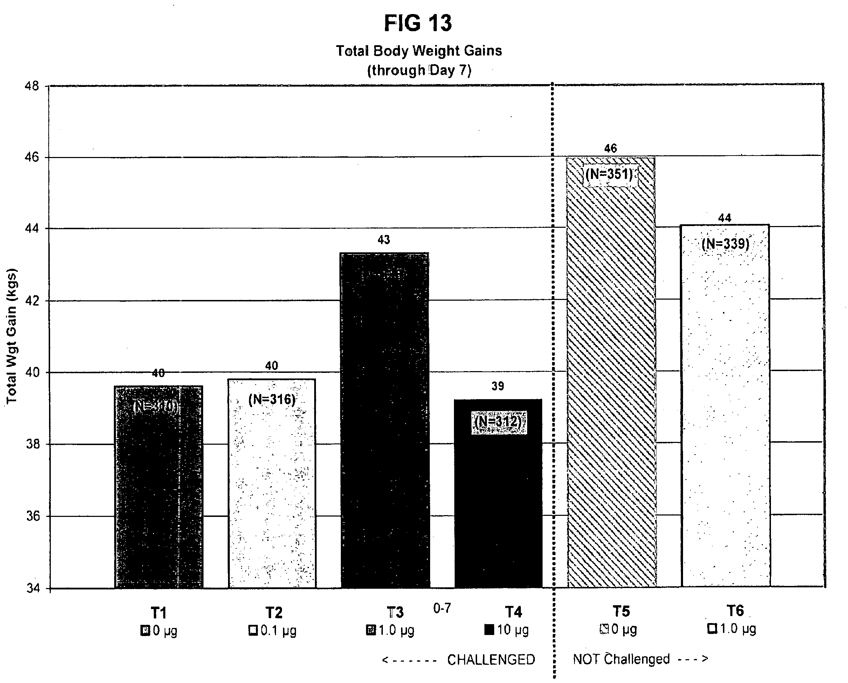

FIG. 13 shows the total average body weight gains observed from Day 0 to Day 7 of the study. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

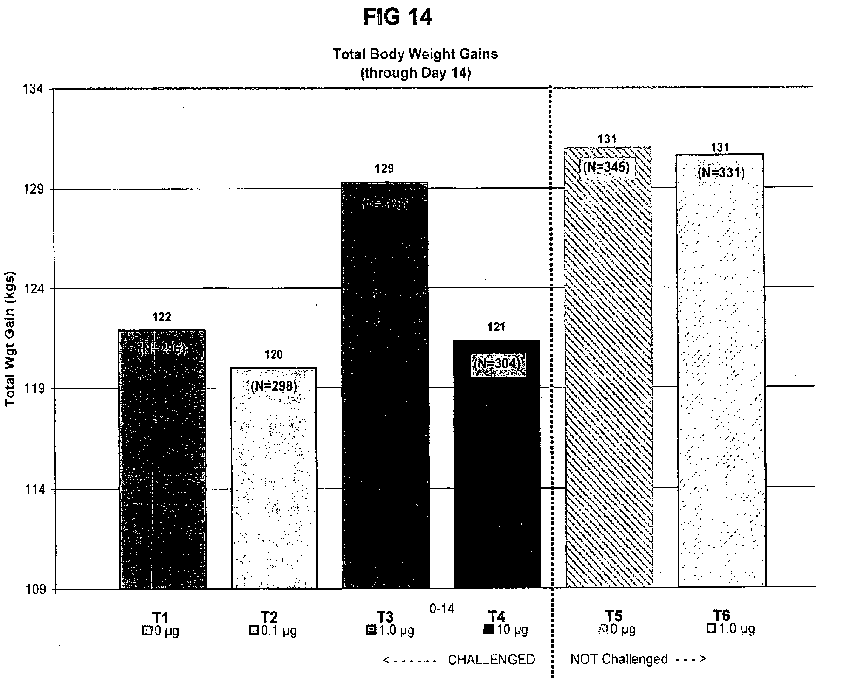

FIG. 14 shows the total average body weight gains observed from Day 0 to Day 14 of the study. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

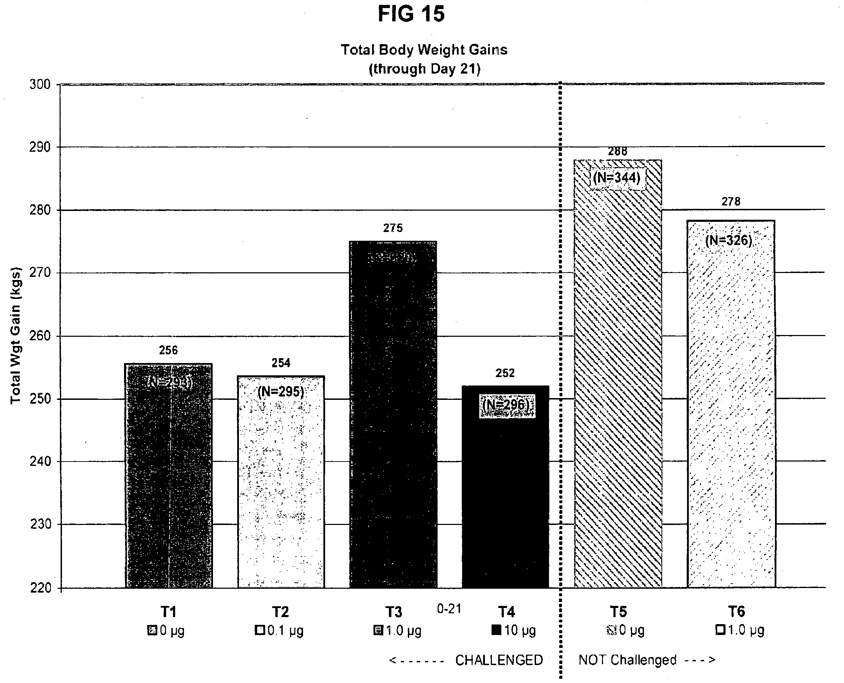

FIG. 15 shows the total average body weight gains observed from Day 0 to Day 21 of the study. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

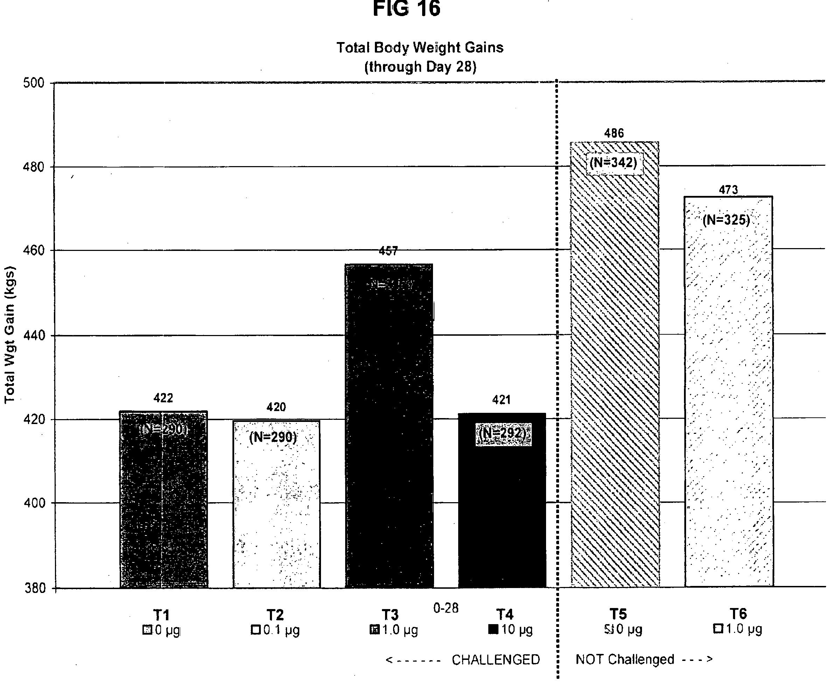

FIG. 16 shows the total average body weight gains observed from Day 0 to Day 28 of the study. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

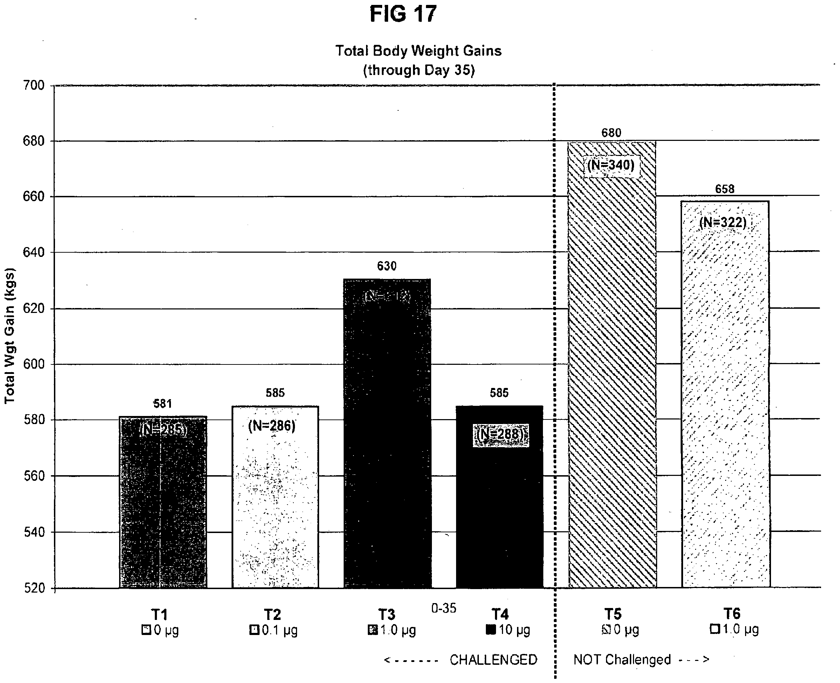

FIG. 17 shows the total average body weight gains observed from Day 0 to Day 35 of the study. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

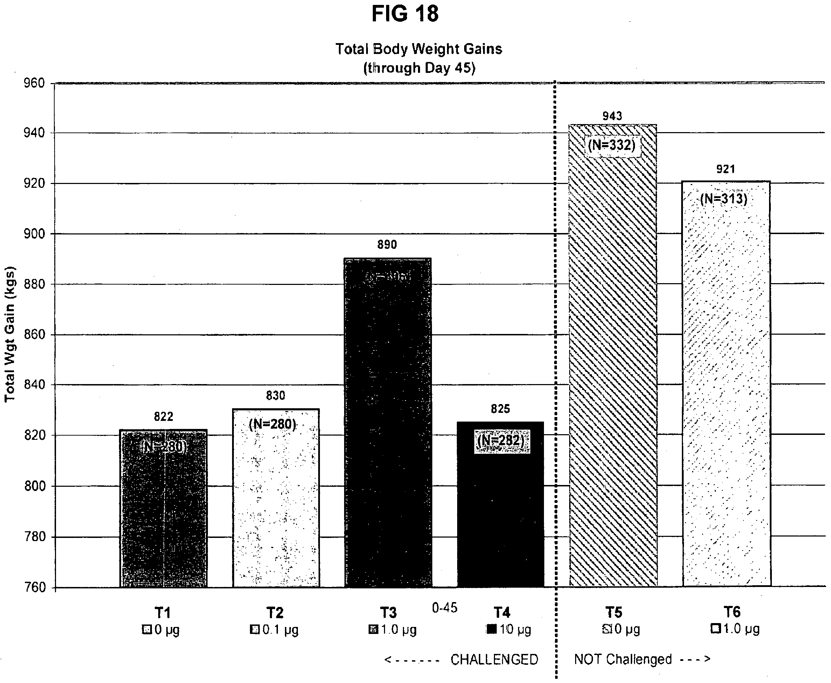

FIG. 18 shows the total average body weight gains observed from Day 0 to Day 45 of the study. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 10.0 µg immunomodulator/egg and E. coli challenged; T5, 0 µg immunomodulator/egg and not challenged; and T6, 1.0 µg immunomodulator/egg and not challenged.

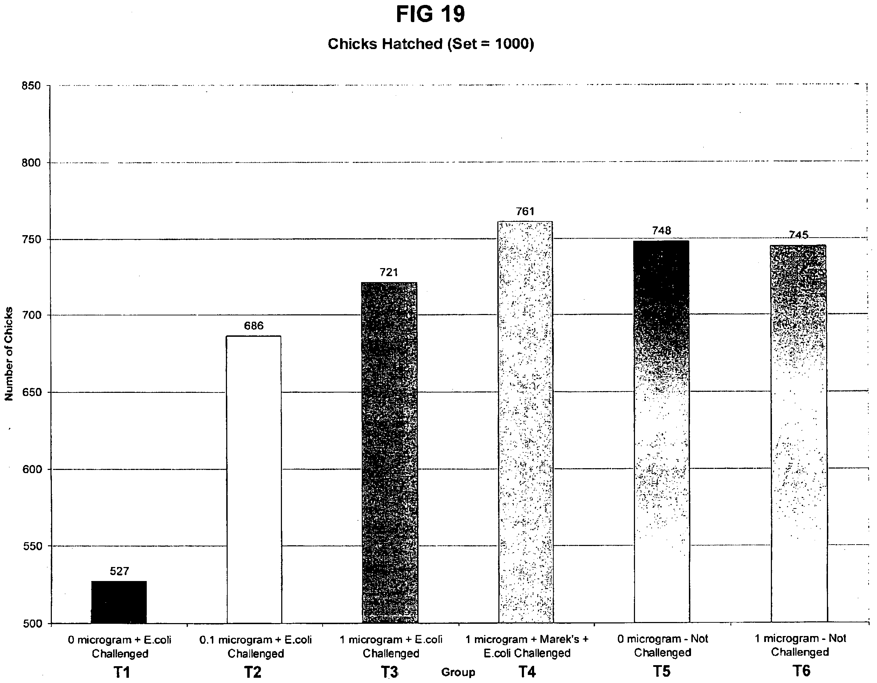

FIG. 19 graphically depicts the number of chickens hatched per study group of embryonated chicken eggs. The study groups analyzed include T1, 0 µg immunomodulator/egg and E. coli challenged; T2, 0.1 µg immunomodulator/egg and E. coli challenged; T3, 1.0 µg immunomodulator/egg and E. coli challenged; T4, 1.0 µg immunomodulator/egg plus 1 dose of Marek's vaccine and E. coli challenged; T5, 0 µg immunomodulator/egg and not challenged; and T6, 1.0 µg immunomodulator/egg and not challenged.

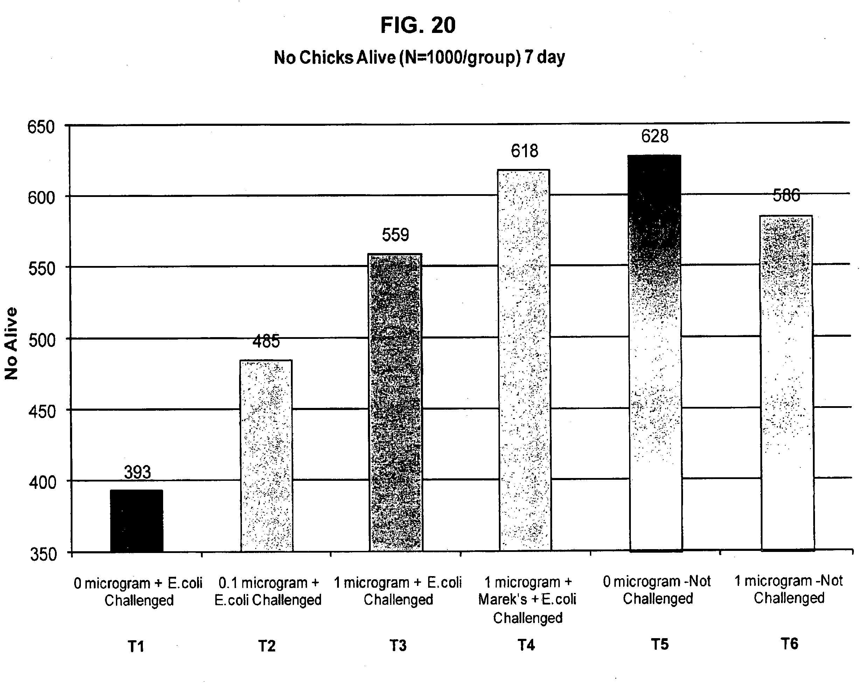

FIG. 20 graphically illustrates the number of live chickens per group on study day 7. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 1.0 µg immunomodulator/egg plus 1 dose of Marek's vaccine and E. coli challenged; T5, 0 µg immunomodulator/egg and not challenged; and T6, 1.0 µg immunomodulator/egg and not challenged.

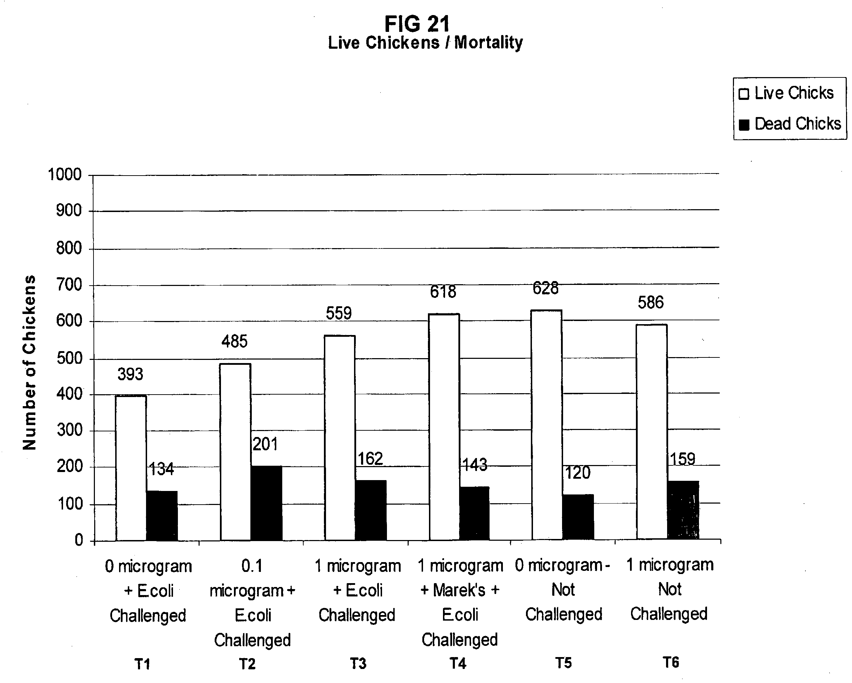

FIG. 21 shows a comparison of live chickens and dead chickens per group on study day 7.

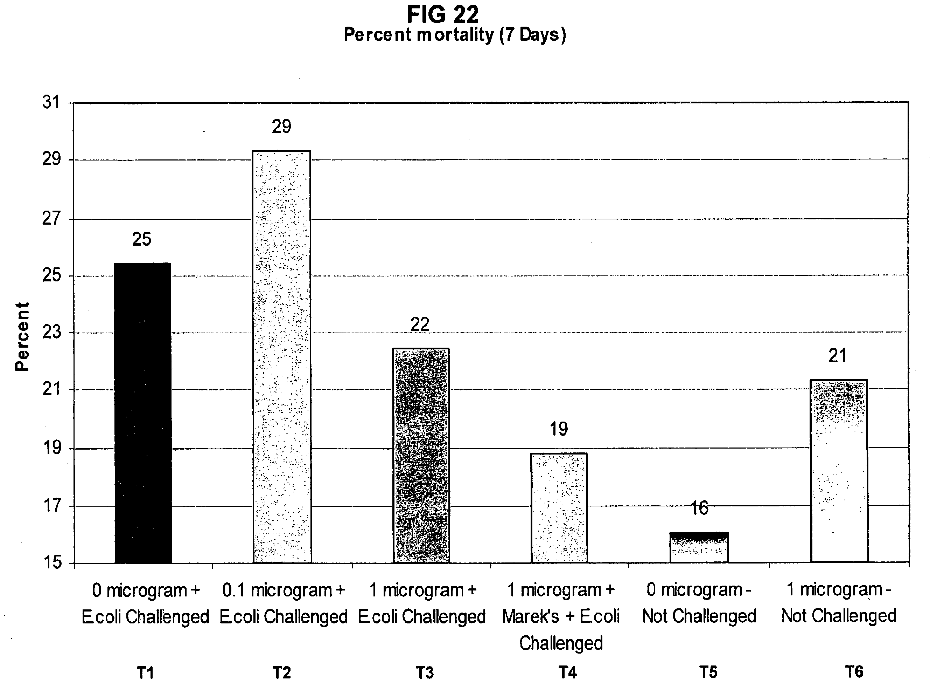

FIG. 22 shows the mortality percentage per study group through day 7 of the study. Key: T1, 0 µg immunomodulator/egg and E. coli challenged; T2, 0.1 µg immunomodulator/egg and E. coli challenged; T3, 1.0 µg immunomodulator/egg and E. coli challenged; T4, 1.0 µg immunomodulator/egg plus 1 dose of Marek's vaccine and E. coli challenged; T5, 0 µg immunomodulator/egg and not challenged; and T6, 1.0 µg immunomodulator/egg and not challenged.

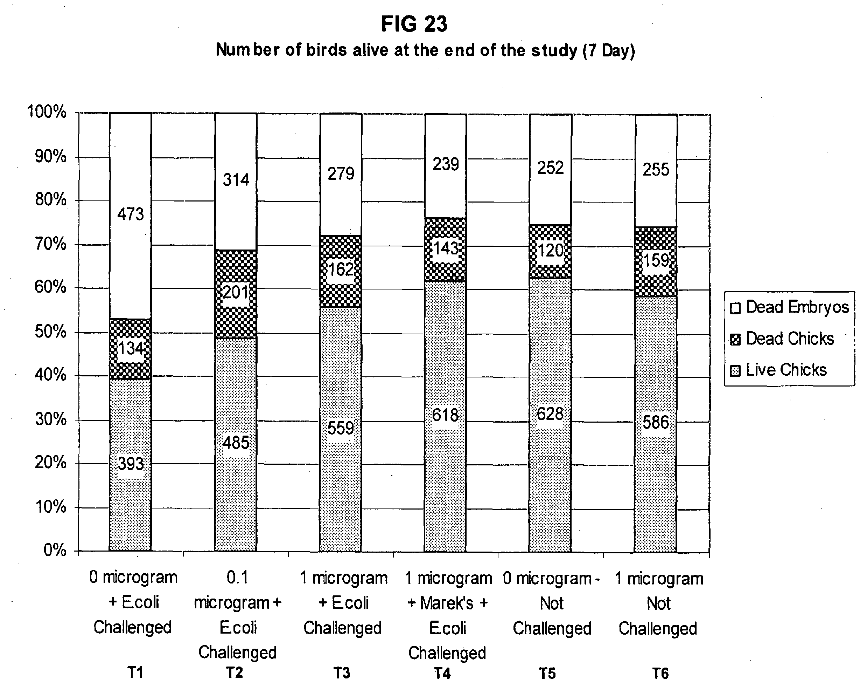

FIG. 23 graphically illustrates the number of birds alive at the end of the study (day 7) for each group including dead embryos, dead chickens, and live chickens (green). Key: T1, 0 µg immunomodulator/egg and E. coli challenged; T2, 0.1 µg immunomodulator/egg and E. coli challenged; T3, 1.0 µg immunomodulator/egg and E. coli challenged; T4, 1.0 µg immunomodulator/egg plus 1 dose of Marek's vaccine and E. coli challenged; T5, 0 µg immunomodulator/egg and not challenged; and T6, 1.0 µg immunomodulator/egg and not challenged.

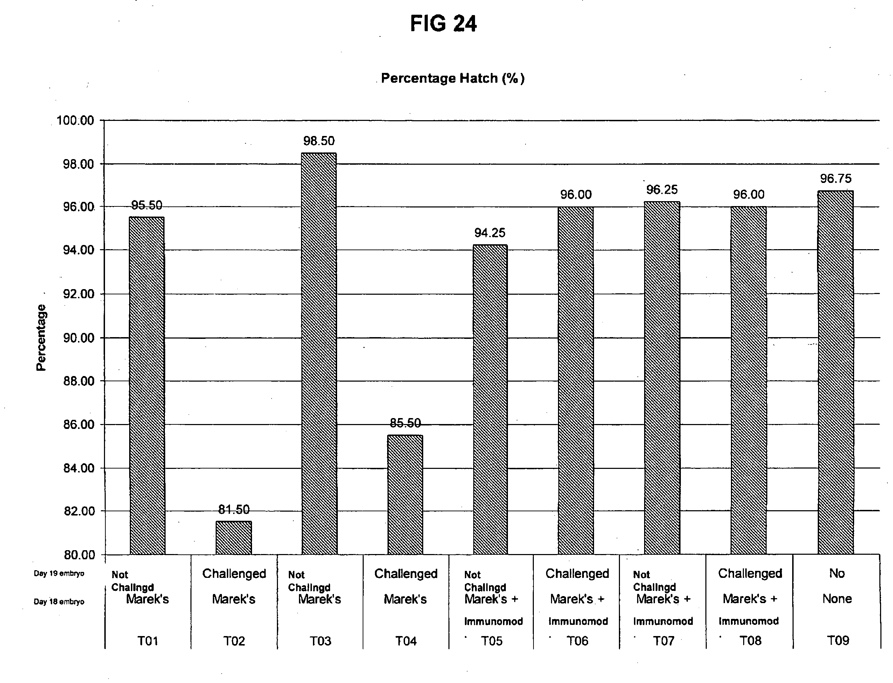

FIG. 24 graphically depicts the percentage of chickens hatched per study group of embryonated chicken eggs. The study groups analyzed include those listed in Table 4.

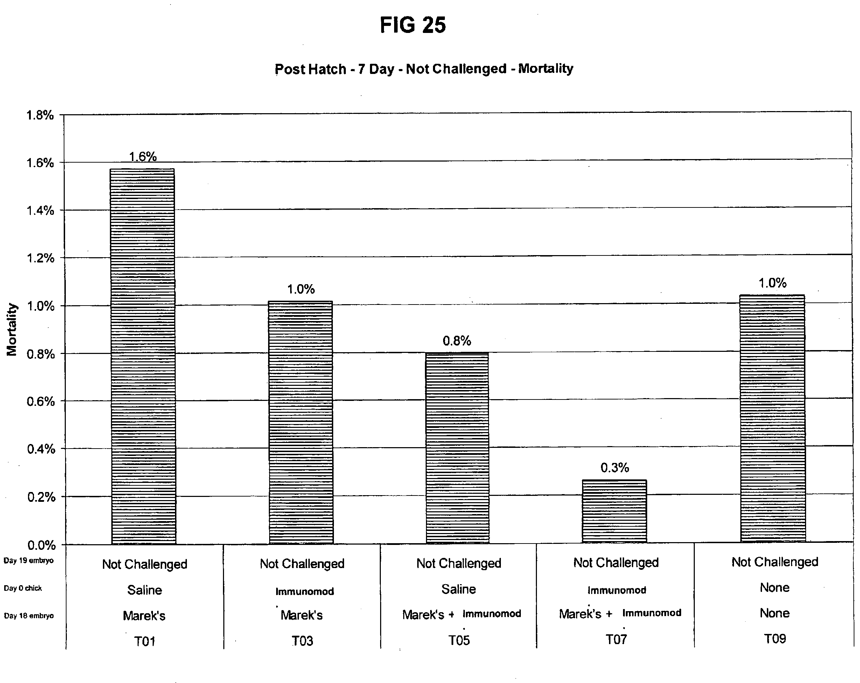

FIG. 25 shows the mortality percentage for study groups not challenged with E. coli after hatch through study day 7. The study groups analyzed include those listed in Table 4.

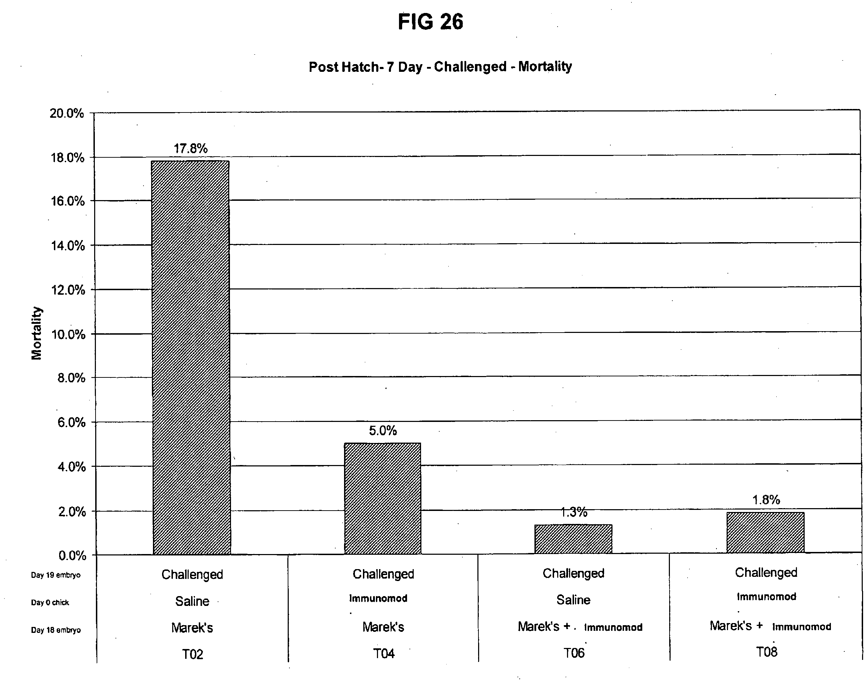

FIG. 26 shows the mortality percentage for study groups challenged with E. coli after hatch through study day 7. The study groups analyzed include those listed in Table 4.

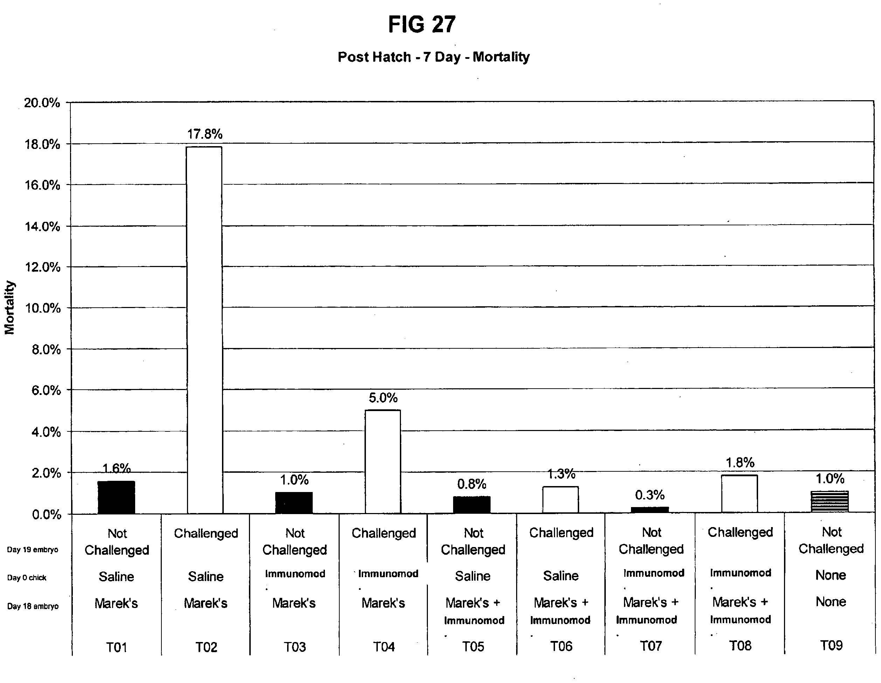

FIG. 27 graphically depicts the mortality percentage for each study group after hatch through study day 7. The study groups analyzed include those listed in Table 4.

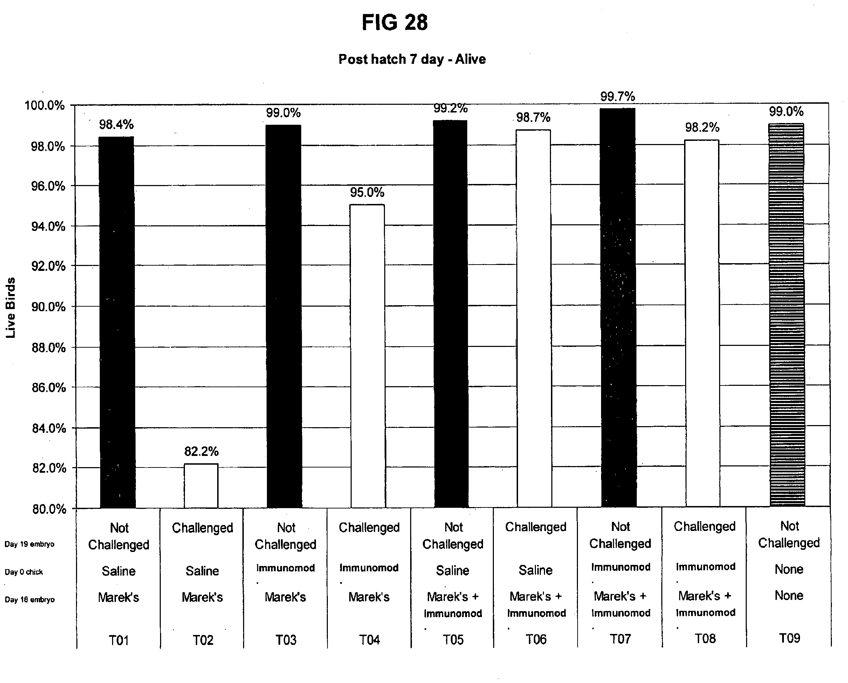

FIG. 28 shows the survival percentage for each study group after hatch through study day 7. The study groups analyzed include those listed in Table 4.

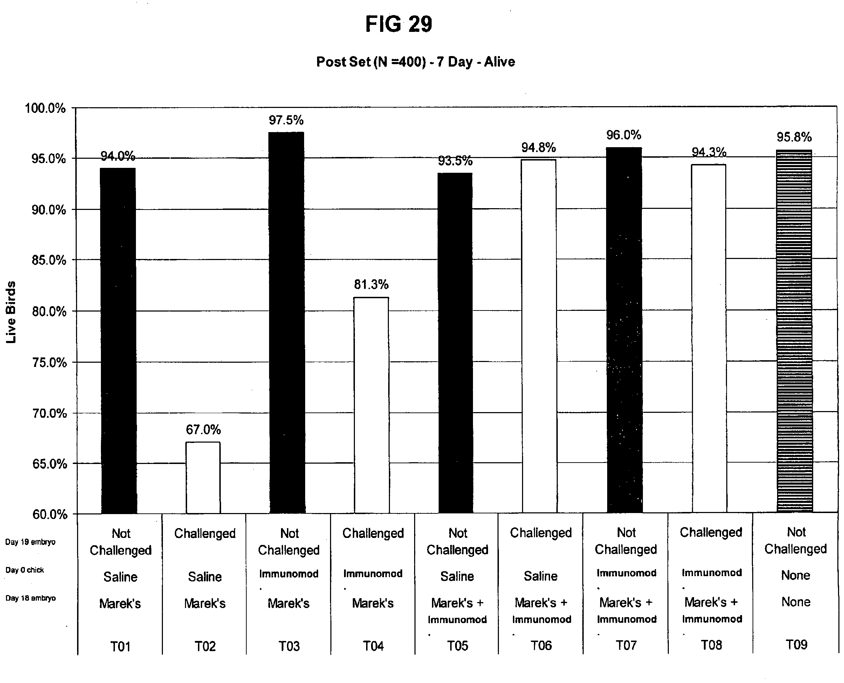

FIG. 29 shows the survival percentage for each study group on study day 7. The study groups analyzed include those listed in Table 4.

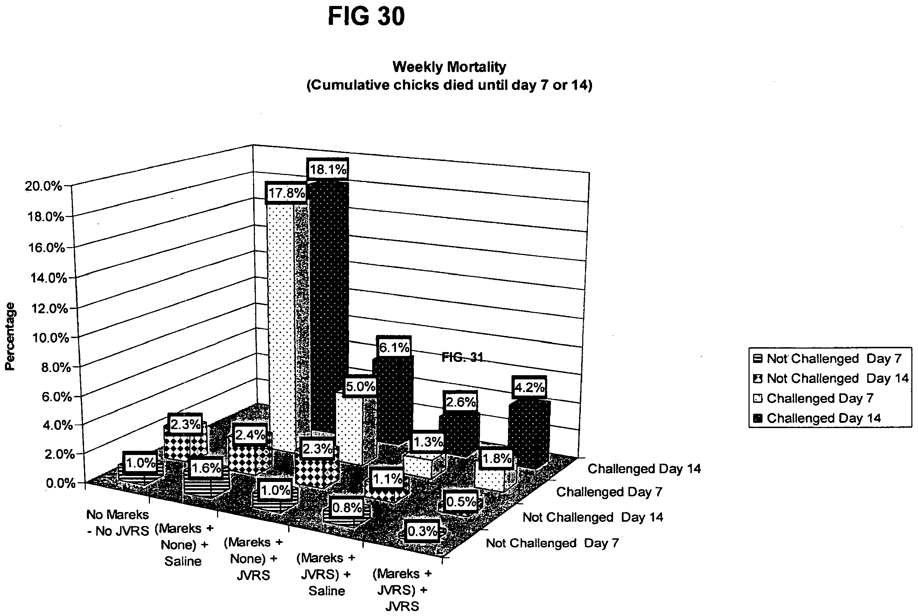

Fig. 30 shows the weekly mortality for each study group through study day 7 and 14. The study groups analyzed include a subgroup of those listed in Table 4.

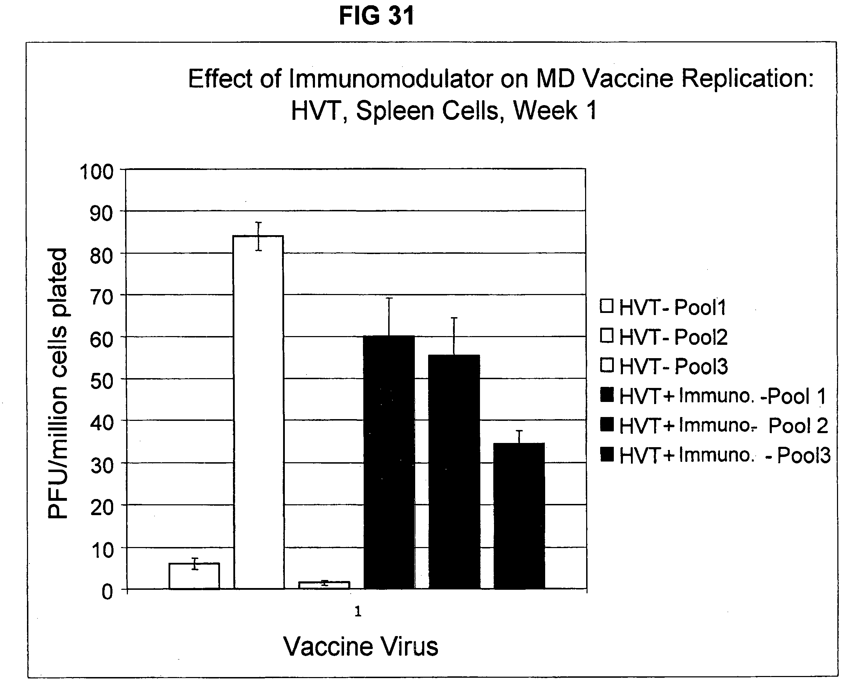

Fig. 31 graphically depics the effect of the immunomodulator on MD vaccine replication of HVT in Spleen Cells in Week one of the study. The study groups analyzed include those listed on Table 5.

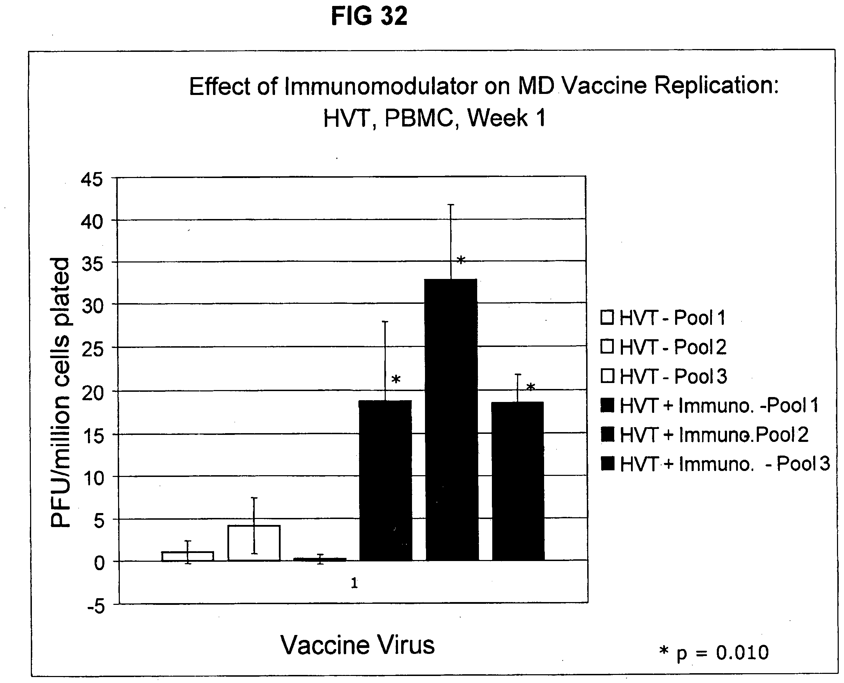

Fig. 32 graphically depics the effect of the immunomodulator on MD vaccine replication of HVT in PMBC in Week one of the study. The study groups analyzed include those listed on Table 5.

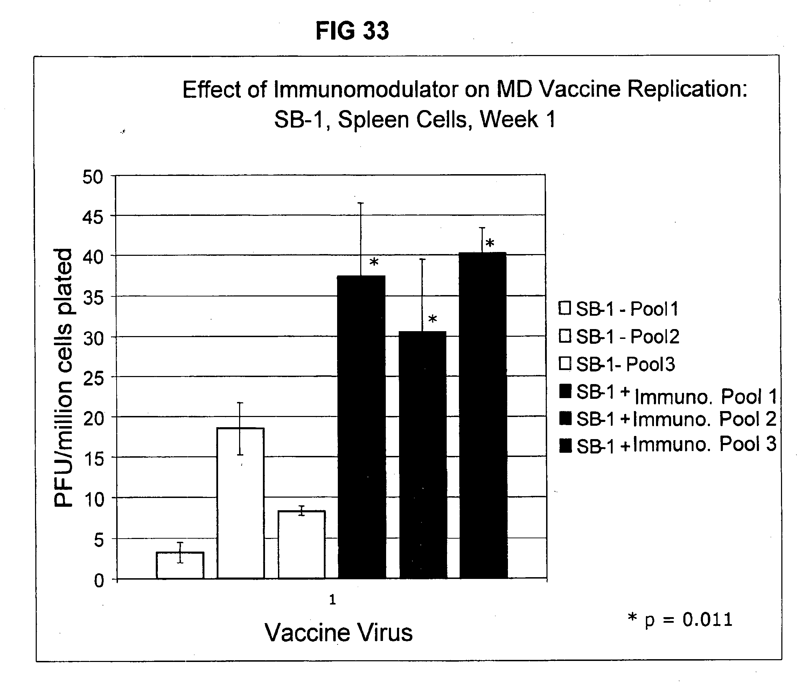

Fig. 33 graphically depics the effect of the immunomodulator on MD vaccine replication of SB-1 in Spleen Cells in Week one of the study. The study groups analyzed include those listed on Table 5.

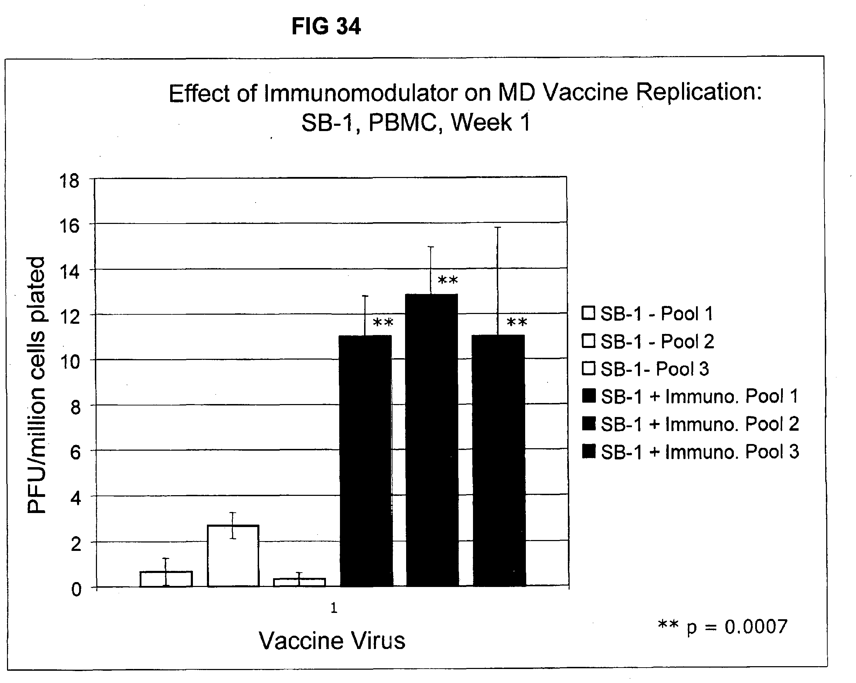

Fig. 34 graphically depics the effect of the immunomodulator on MD vaccine replication of SB-1 in PMBC in Week one of the study. The study groups analyzed include those listed on Table 5.

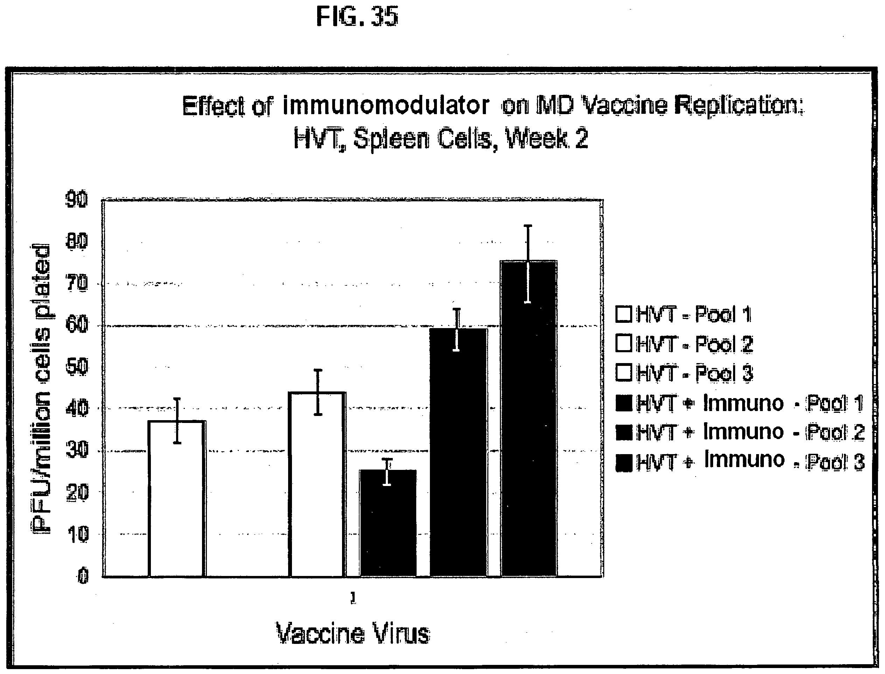

Fig. 35 graphically depics the effect of the immunomodulator on MD vaccine replication of HVT in Spleen Cells in Week two of the study. The study groups analyzed include those listed on Table 5.

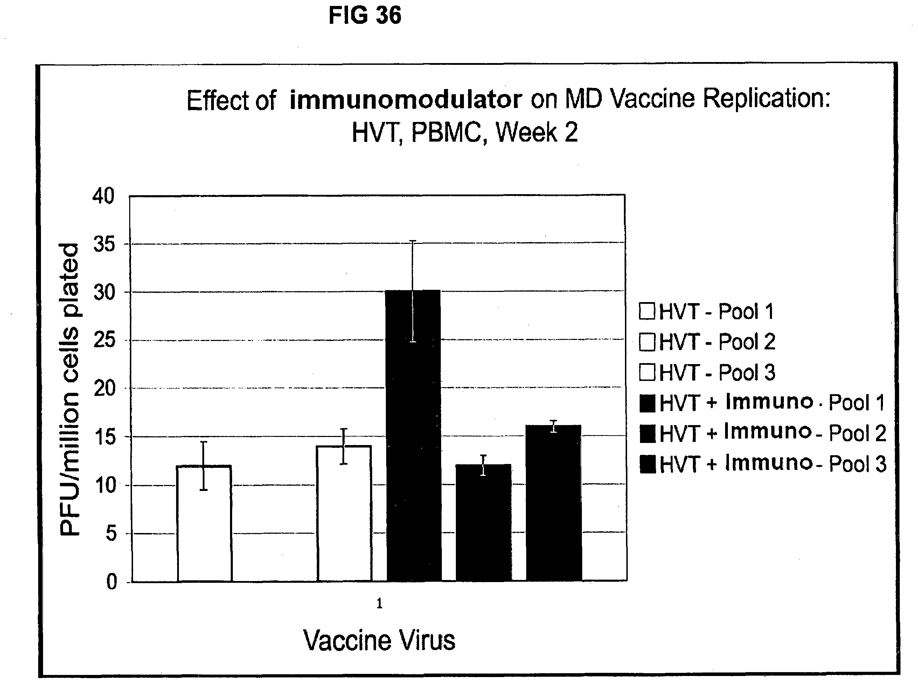

Fig. 36 graphically depics the effect of the immunomodulator on MD vaccine replication of HVT in PMBC in Week two of the study. The study groups analyzed include those listed on Table 5.

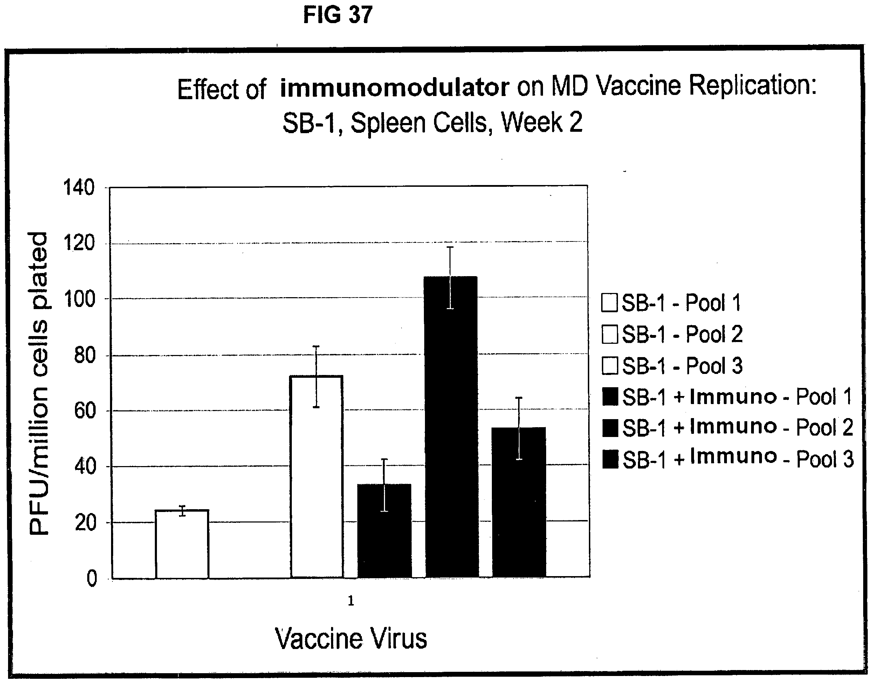

Fig. 37 graphically depics the effect of the immunomodulator on MD vaccine replication of SB-1 in Spleen Cells in Week two of the study. The study groups analyzed include those listed on Table 5.

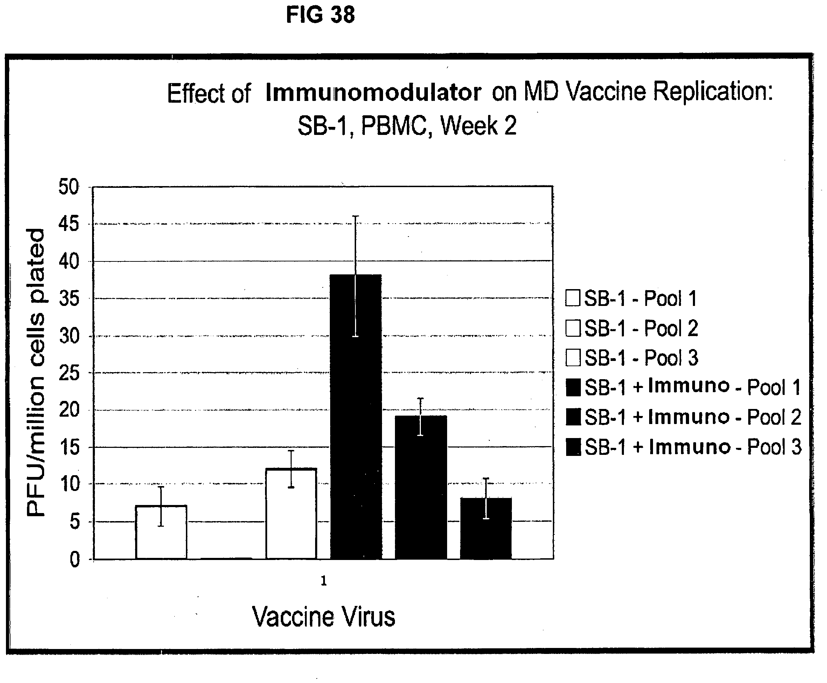

Fig. 38 graphically depics the effect of the immunomodulator on MD vaccine replication of SB-1 in PMBC in Week two of the study. The study groups analyzed include those listed on Table 5.

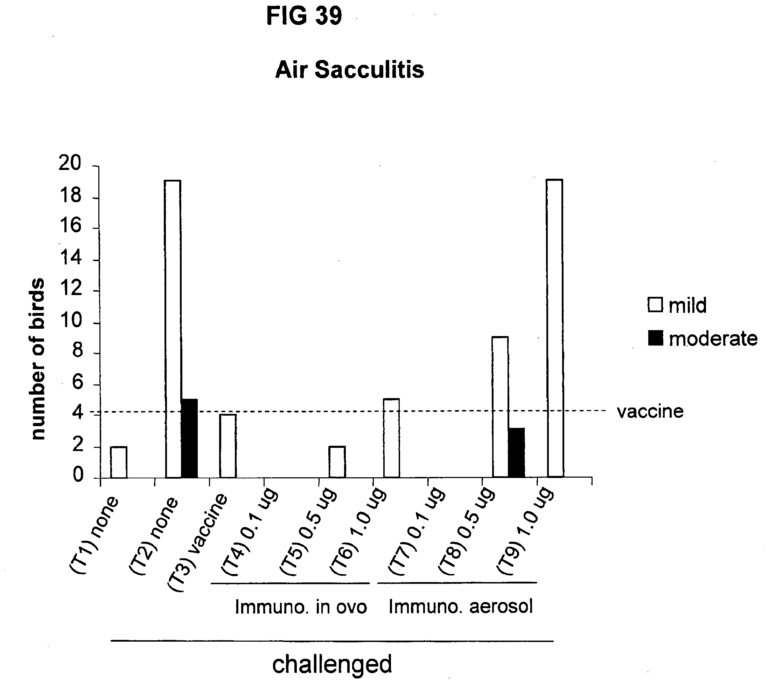

Fig. 39 graphically depics the reduction in air sacculities in the chicks that received the immunomodulator in ovo, were vaccinated against ND and were challenged. The study groups analyzed include those listed in Table 7.

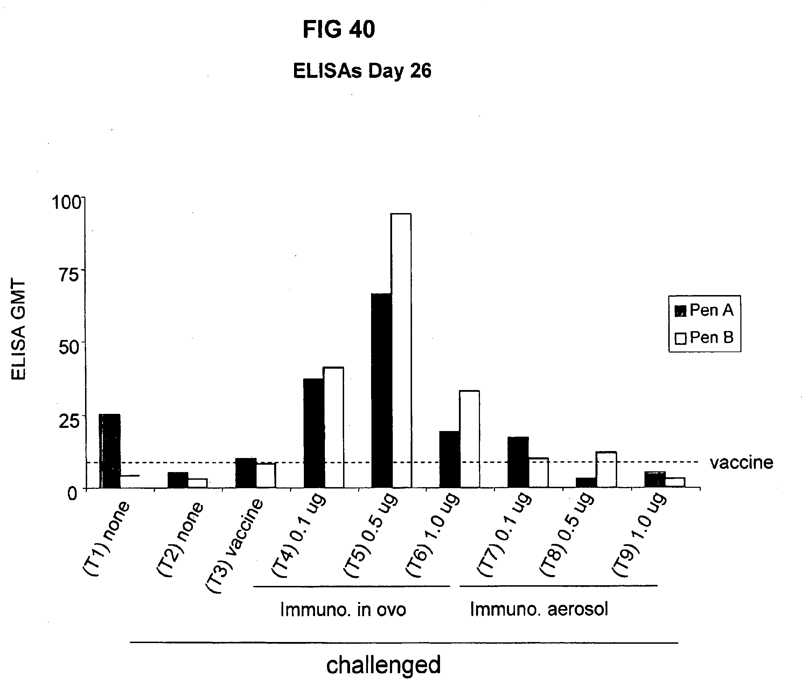

Fig. 40 graphically depics the ELISAs at day 26 for the study groups listed in Table 7.

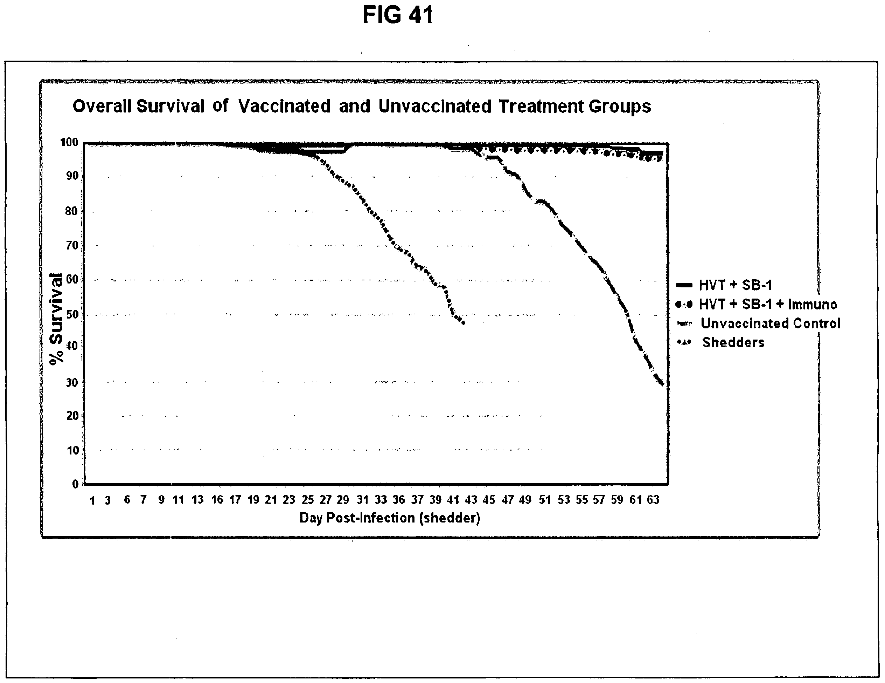

Fig. 41 graphically depics the survival rates for the chicks in the study groups listed in Table 8.

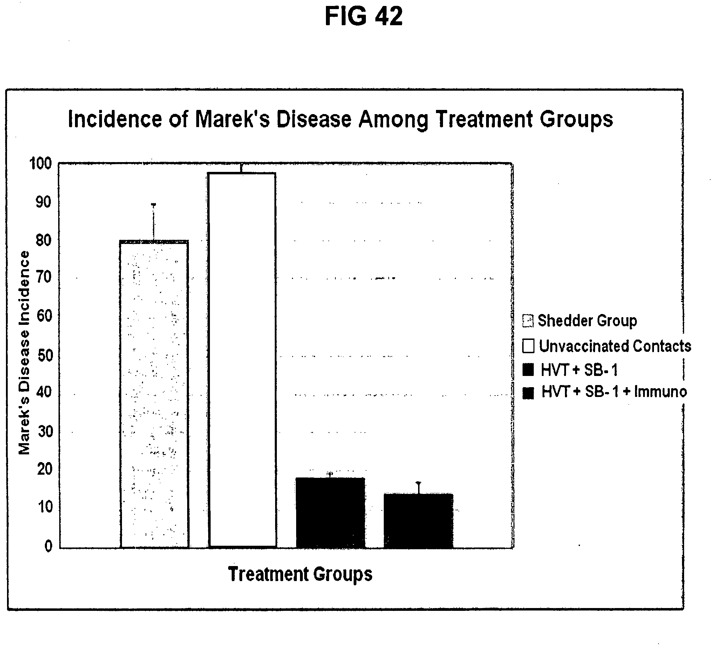

Fig. 42 graphically depics the incidence of Marek's disease among the study groups listed in Table 8.

### DETAILED DESCRIPTION OF THE INVENTION

The method of eliciting an immune response in a member of the avian species of the present disclosure includes administering to the member of the avian species an effective amount of an immunomodulator composition to elicit an immune response in the member of the avian species. The immunomodulator composition of the invention includes a cationic liposome delivery vehicle and a DNA plasmid that does not code for an immunogen for use in preventing a reduction in survival rate when co-administered with a vaccine of a chicken hatched from an embryonated chicken egg, by administration *in ovo* to the egg of an effective amount of 0.05 to 10 micrograms, preferably 0.1 to 5 micrograms, of the immunomodulator composition, wherein the vaccine is a vaccine used for protection against Marek's disease virus (MDV). In particular, the immunomodulator elicits a non-antigen-specific immune response that may be further enhanced with administration of at least one biological agent such as a vaccine.

The methods provide new treatment strategies for protecting the avian species from infectious diseases and treating populations having infectious disease. In addition, by using the immunomodulator composition of the present disclosure there is no reduction in hatchability or survival rate after hatch when the immunomodulator is administered in ovo and there is no reduction in hatchability or survival rate when co-administered with a vaccine. Further, pre-administration of the immunomodulator prior to the administration of a vaccine further enhances the non-antigen-specific immune response. The methods of the present disclosure also allow the safe use of vaccines previously only available for administration after hatch to be used in ovo. In addition, the method of the present disclosure allows the combination of more than one vaccine with the immunomodulator composition. Finally, the method of the present disclosure provides longer protection against a disease when the immunomodulator is used in combination with a vaccine.

The invention is defined by the appended claims.

### I. Composition

### a. Immunomodulator

In one embodiment of the disclosure, the immunomodulator composition includes a liposome delivery vehicle and at least one nucleic acid molecule, as described in U.S. Patent No. 6,693,086.

A suitable liposome delivery vehicle comprises a lipid composition that is capable of delivering nucleic acid molecules to the tissues of the treated subject. A liposome delivery vehicle is preferably capable of remaining stable in a subject for a sufficient amount of time to deliver a nucleic acid molecule and/or a biological agent. In one embodiment, the liposome delivery vehicle is stable in the recipient subject for at least about 30 minutes. In another embodiment, the liposome delivery vehicle is stable in the recipient subject for at least about 1 hour. In yet another embodiment, the liposome delivery vehicle is stable in the recipient subject for at least about 24 hours.

A liposome delivery vehicle of the present disclosure comprises a lipid composition that is capable of fusing with the plasma membrane of a cell to deliver a nucleic acid molecule into a cell. In one embodiment, when delivered a nucleic acid: liposome complex of the present disclosure is at least about 1 picogram (pg) of protein expressed per milligram (mg) of total tissue protein per microgram (µg) of nucleic acid delivered. In another embodiment, the transfection efficiency of a nucleic acid: liposome complex is at least about 10 pg of protein expressed per mg of total tissue protein per µg of nucleic acid delivered; and in yet another embodiment, at least about 50 pg of protein expressed per mg of total tissue protein per µg of nucleic acid delivered. The transfection efficiency of the complex may be as low as 1 femtogram (fg) of protein expressed per mg of total tissue protein per µg of nucleic acid delivered, with the above amounts being more preferred.

A preferred liposome delivery vehicle of the present invention is between about 100 and 500 nanometers (nm), in another embodiment, between about 150 and 450 nm and in yet another embodiment, between about 200 and 400 nm in diameter.

Suitable liposomes include any liposome, such as those commonly used in, for example, gene delivery methods known to those of skill in the art. Preferred liposome delivery vehicles comprise multilamellar vesicle (MLV) lipids and extruded lipids. Methods for preparation of MLV's are well known in the art. More preferred liposome delivery vehicles comprise liposomes having a polycationic lipid composition (i.e., cationic liposomes) and/or liposomes having a cholesterol backbone conjugated to polyethylene glycol. Exemplary cationic liposome compositions include, but are not limited to, N-[1-(2,3-dioleyloxy)propyl]-N,N,N-trimethylammonium chloride (DOTMA) and cholesterol, N-[1-(2,3-dioleoyloxy)propyl]-N,N,N-trimethylammonium chloride (DOTAP) and cholesterol, 1-[2-(oleoyloxy)ethyl]-2-oleyl-3-(2-hydroxyethyl)imidazolinium choloride (DOTIM) and cholesterol, dimethyldioctadecylammonium bromide (DDAB) and cholesterol, and combinations thereof. A most preferred liposome composition for use as a delivery vehicle includes DOTIM and cholesterol.

A suitable nucleic acid molecule includes any nucleic acid sequence such as coding or non-coding sequence, and DNA or RNA. Coding nucleic acid sequences encode at least a portion of a protein or peptide, while non-coding sequence does not encode any portion of a protein or peptide. According to the present disclosure, "non-coding" nucleic acids can include regulatory regions of a transcription unit, such as a promoter region. The term, "empty vector" can be used interchangeably with the term "non-coding", and particularly refers to a nucleic acid sequence in the absence of a protein coding portion, such as a plasmid vector without a gene insert. Expression of a protein encoded by the nucleic acid molecule is not required for elicitation of a non-antigen-specific immune response; therefore the nucleic acid molecule does not necessarily need to be operatively linked to a transcription control sequence. However, further advantages may be obtained (i.e., antigen-specific and enhanced immunity) by including in the composition nucleic acid sequence (DNA or RNA) which encodes an immunogen and/or a cytokine.

Complexing a liposome with a nucleic acid molecule may be achieved using methods standard in the art or as described in U.S. Patent No. 6,693,086. A suitable concentration of a nucleic acid molecule to add to a liposome includes a concentration effective for delivering a sufficient amount of nucleic acid molecule into a subject such that a systemic immune response is elicited. When the nucleic acid molecule encodes an immunogen or a cytokine, a suitable concentration of nucleic acid molecule to add to a liposome includes a concentration effective for delivering a sufficient amount of nucleic acid molecule into a cell such that the cell can produce sufficient immunogen and/or cytokine protein to regulate effector cell immunity in a desired manner. In one embodiment, from about 0.1 µg to about 10 µg of nucleic acid molecule is combined with about 8 nmol liposomes, in another embodiment, from about 0.5 µg to about 5 µg of nucleic acid molecule is combined with about 8 nmol liposomes, and in yet another embodiment, about 1.0 µg of nucleic acid molecule is combined with about 8 nmol liposomes. In one embodiment, the ratio of nucleic acids to lipids (µg nucleic acid: nmol lipids) in a composition is at least about 1:1 nucleic acid: lipid by weight (i.e., 1 µg nucleic acid: 1 nmol lipid), and in another embodiment, at least about 1:5, and in yet another embodiment, at least about 1:10, and in a further embodiment at least about 1:20. Ratios expressed herein are based on the amount of cationic lipid in the composition, and not on the total amount of lipid in the composition. In another embodiment, the ratio of nucleic acids to lipids in a composition of the disclosure is from about 1:1 to about 1:64 nucleic acid: lipid by weight; and in another embodiment, from about 1:5 to about 1:50 nucleic acid: lipid by weight; and a further embodiment, from about 1:10 to about 1:40 nucleic acid: lipid by weight; and in yet another embodiment, from about 1:15 to about 1:30 nucleic acid: lipid by weight. Another ratio of nucleic acid: lipid is from about 1:8 to 1:16, with about 1:8 to 1:32 being preferred.

### b. Biological agent

In another embodiment of the disclosure, the immunomodulator includes a liposome delivery vehicle, a nucleic acid molecule, and at least one biological agent.

Suitable biological agents are agents that are effective in preventing or treating avian disease. Such biological agents include immune enhancer proteins, immunogens, vaccines or any combination thereof. Suitable immune enhancer proteins are those proteins known to enhance immunity. By way of a non-limiting example, a cytokine, which includes a family of proteins, is a known immunity enhancing protein family. Suitable immunogens are proteins which elicit a humoral and/or cellular immune response such that administration of the immunogen to a subject mounts an immunogen-specific immune response against the same or similar proteins that are encountered within the tissues of the subject. An immunogen may include a pathogenic antigen expressed by a bacterium, a virus, a parasite or a fungus. Preferred antigens include antigens which cause an infectious disease in a subject. According to the present disclosure, an immunogen may be any portion of a protein, naturally occurring or synthetically derived, which elicits a humoral and/or cellular immune response. As such, the size of an antigen or immunogen may be as small as about 5-12 amino acids and as large as a full length protein, including sizes in between. The antigen may be a multimer protein or fusion protein. The antigen may be purified peptide antigens derived from native or recombinant cells. The nucleic acid sequences of immune enhancer proteins and immunogens are operatively linked to a transcription control sequence, such that the immunogen is expressed in a tissue of a subject, thereby eliciting an immunogen-specific immune response in the subject, in addition to the non-specific immune response.

In another embodiment of the disclosure, the biological agent is a vaccine. The vaccine may include a live, infectious, viral vaccine or a killed, inactivated, viral vaccine. In one embodiment, one or more vaccines, live or killed viral vaccines, may be used in combination with the immunomodulator composition of the present disclosure. Suitable vaccines include those known in the art for the avian species. Exemplary vaccines, without limitation, include those used in the art for protection against Marek's disease virus (MDV), New Castle disease virus (NDV), chick anemia virus (CAV), infectious bursal disease virus (IBDV), infectious bronchitis virus (IBV), turkey herpesvirus (HVT), infectious laryngotracheitis virus (ILTV), avian encephalomyelitis virus (AEV), fowlpox virus (FPV), fowl cholera, avian influenza virus (AIV), reovirus, avian leucosis virus (ALV), reticuloendotheliosis virus (REV), avian adenovirus and hemorrhagic enteritis virus (HEV), coccidia, and other diseases known in the art. In another example, the vaccine may be a vaccine as described by US Patent Nos. 5,427,791, 6,048,535, and 6,406,702. As defined in the appended claims, a vaccine for the protection of Marek's disease is used in combination with the immunomodulator composition of the present invention.

### II. Methods

### a. Methods of immune stimulation

In one embodiment of the disclosure, an immune response is elicited in a member of the avian species by administering an effective amount of an immunomodulator composition to the member of the avian species. The effective amount is sufficient to elicit an immune response in the member of the avian species. The immunomodulator includes a liposome delivery vehicle and a nucleic acid molecule.

In one embodiment, the effective amount of the immunomodulator is from about 0.05 micrograms to about 10 micrograms. In another embodiment, the effective amount of the immunomodulator is from about 0.1 micrograms to about 5 micrograms.

In another embodiment of the disclosure, an immune response is elicited in a member of the avian species by administering an effective amount of an immunomodulator composition that includes a liposome delivery vehicle, an isolated nucleic acid molecule, and a biological agent. It is contemplated that the biological agent may be co-administered with the immunomodulator or independently thereof. Independent administration may be prior to or after administration of the immunomodulator. It is also contemplated that more than one administration of the immunomodulator or biological agent may be used to extend enhanced immunity. Furthermore, more than one biological agent may be co-administered with the immunomodulator, administered prior to the immunomodulator, or administered after administration of the immunomodulator as described in the Examples herein. In one embodiment, the effective amount of the immunomodulator is from about 0.05 micrograms to about 5 micrograms of liposome delivery vehicle and isolated nucleic acid molecule and from about 300 to about 30000 TCID50 virus biological agent. In another embodiment, the effective amount of the immunomodulator is from about 0.1 micrograms to about 3 micrograms of liposome delivery vehicle and isolated nucleic acid molecule and from about 100 to about 1000 EID50 virus biological agent.

### b. Diseases

The methods of the disclosure elicit an immune response in a subject such that the subject is protected from a disease that is amenable to elicitation of an immune response. As used herein, the phrase "protected from a disease" refers to reducing the symptoms of the disease; reducing the occurrence of the disease, and reducing the severity of the disease. Protecting a subject can refer to the ability of a therapeutic composition of the present disclosure, when administered to a subject, to prevent a disease from occurring and/or to cure or to alleviate disease symptoms, signs, or causes. As such, to protect a member of the avian species from a disease includes both preventing disease occurrence (prophylactic treatment) and treating a member of the avian species that has a disease (therapeutic treatment). In particular, protecting a subject from a disease is accomplished by eliciting an immune response in the member of the avian species by inducing a beneficial or protective immune response which may, in some instances, additionally suppress, reduce, inhibit, or block an overactive or harmful immune response. The term "disease" refers to any deviation from the normal health of a member of the avian species and includes a state when disease symptoms are present, as well as conditions in which a deviation (e.g., infection, gene mutation, genetic defect, etc.) has occurred, but symptoms are not yet manifested.

Methods of the disclosure may be used for the prevention of disease, stimulation of effector cell immunity against disease, elimination of disease, alleviation of disease, and prevention of a secondary disease resulting from the occurrence of a primary disease.

Methods of the disclosure include administering the composition to protect against infection of a wide variety of pathogens. The composition administered may or may not include a specific antigen to elicit a specific response to. It is contemplated that the methods of the disclosure will protect the recipient subject from disease resulting from infectious microbial agents including, without limitation, viruses, bacteria, fungi, and parasites. Exemplary viral infectious diseases, without limitation, include those resulting from infection with chicken infectious anemia virus (CIAV), Marek's disease virus (MDV), herpesvirus chicken (HCV), herpesvirus turkey (HTV), infectious bursal disease virus (IBDV), Newcastle disease virus (NDV), infectious bronchitis virus (IBV), infectious laryngotracheitis virus (ILTV), paramyxovirus type 3, avian encephalomyelitis (AEV), Fowlpox virus (FPV), fowl cholera, avian influenza virus (AIV), reovirus, avian leukosis virus (ALV), reticuloendotheliosis virus (REV), avian adenovirus, hemorrhagic enteritis virus (HEV), pneumovirus, pigeon pox virus, recombinants thereof, and other viruses known in the art. Exemplary bacterial infections, without limitation, include those resulting from infection with gram positive or negative bacteria and fungi such as *Escherichia coli, Mycoplasma gallisepticum, Mycoplasma meleagridis, Mycoplasma synoviae, Bordetella Sp, Pasteurella multocida, Clostridium perfringens, Clostridium colinum, Campylobacter jejuni, Salmonella sp, Salmonella enteritidis, Salmonella pullorum, Salmonella gallinarum, Clostridium botulinum, Hemophilus gallinarum, Erysipelothrix insidiosa, riemerella anatipestifer (RA),* and other bacteria known in the art. Exemplary fungi or mold infection, without limitation, include those resulting from infection with *Aspergillus fumigates, Aspergillus flavus, Candida albicans*, and other infectious fungi or mold known in the art. Exemplary disease conditions, without limitation, include those resulting from toxins from gram positive or negative bacteria and fungi such as clostridium perfringesns toxins, clostridium botulinum toxin, E.coli enterotoxin, staphylococcus toxins, pasteurella leukotoxin, and fusarium mycotoxins and other toxins known in the art. Exemplary parasites include, without limitation, *Eimeria meleagridis, coccidia sp, Ascaridia galli, Heterakis gallinae, Capillaria annulata, Capillaria contorta, Capillaria obsignata, Syngamus trachea*, and other parasites known in the art.

### c. Subjects

The methods of the disclosure may be administered to any subject or member of the avian species, whether domestic or wild. In particular, it may be administered to those subjects that are commercially reared for breeding, meat or egg production. Suitable avian subjects, without limitation, include chickens, turkeys, geese, ducks, pheasants, quail, pigeons, ostriches, caged birds, birds in zoological collections and aviaries and the like. In one embodiment of the disclosure, the member of the avian species is selected from the group consisting of chicken or turkey. A skilled artisan will appreciate that the methods of the disclosure will be largely beneficial to birds reared in high density brooder houses, such as broiler and layer chickens, since they are especially vulnerable to environmental exposure to infectious agents.

### d. Administration

A variety of administration routes are available. The particular mode selected will depend, of course, upon the particular biological agents selected, the age and general health status of the subject, the particular condition being treated and the dosage required for therapeutic efficacy. The methods of this disclosure may be practiced using any mode of administration that produces effective levels of an immune response without causing clinically unacceptable adverse effects. The compositions may conveniently be presented in unit dosage form and may be prepared by any of the methods well known in the art.

Vaccination of birds can be performed at any age. Normally, vaccinations are performed to 18 day old embryo (in ovo) and above for a live microorganism and 3 weeks and older for an inactivated microorganism or other type of vaccine. For in ovo vaccination, vaccination can be performed in the last quarter of embryo development. The vaccine may be administered subcutaneously, feather follicle, by spray, orally, intraocularly, intratracheally, nasally, in ovo or by other methods known in the art. The oral vaccine may be administered in the drinking water. Further, it is contemplated that the methods of the disclosure may be used based on routine vaccination schedules. According to the appended claims, the immunomodulator composition of the present invention is administered in ovo. In another embodiment, the immunomodulator composition is administered as a spray after challenge with E. coli..

Other delivery systems may include time-release, delayed release or sustained release delivery systems. Such systems can avoid repeated administrations of the compositions therefore increasing convenience. Many types of release delivery systems are available and known to those of ordinary skill in the art. They include polymer based systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent No. 5,075,109. Delivery systems also include non-polymer systems that are lipids including sterols such as cholesterol, cholesterol esters and fatty acids or neutral fats such as mono-di and tri-glycerides; hydrogel release systems; sylastic systems; peptide based systems; wax coatings; compressed tablets using convention binders and excipients; partially fused implants; and the like. Specific examples include, but are not limited to erosional systems in which an agent of the disclosure is contained in a form within a matrix such as those described in U.S. Patent Nos. 4,452,775, 4,675,189 and 5,736,152, and diffusional systems in which an active component permeates at a controlled rate from a polymer such as described in U.S. Patent Nos. 3, 854,480, 5,133,974 and 5,407,686. In addition, pump-based hardware delivery systems can be used, some of which are adapted for implantation.

As various changes could be made in the above composition, products and methods without departing from the scope of the invention, it is intended that all matter contained in the above description and in the examples given below, shall be interpreted as illustrative and not in a limiting sense.

### DEFINITIONS

The term "effective amount" refers to the amount necessary or sufficient to realize a desired biologic effect. For example, an effective amount of immunomodulator for treating or preventing an infectious disease is that amount necessary to cause the development of an antigen specific immune response upon exposure to the microbe, thus causing a reduction in the amount of microbe within the subject and preferably to the eradication of the microbe. The effective amount for any particular application can vary depending on such factors as the disease or condition being treated, the size of the subject, or the severity of the disease or condition. One of ordinary skill in the art can empirically determine the effective amount of immunomodulator without necessitating undue experimentation.

The term "cytokine" refers to an immune enhancing protein family. The cytokine family includes hematopoietic growth factor, interleukins, interferons, immunoglobulin superfamily molecules, tumor necrosis factor family molecules and chemokines (i.e. proteins that regulate the migration and activation of cells, particularly phagocytic cells). Exemplary cytokines include, without limitation, interleukin-2 (IL-2), interleukin -12 (IL12), interleukin-15 (IL-15), interleukin-18 (IL-18), interferon-a (IFNα) interferon-a (IFNα), and interferon-a (IFNα).

The term "elicit" can be used interchangeably with the terms activate, stimulate, generate or upregulate.

The term "eliciting an immune response" in a subject refers to specifically controlling or influencing the activity of the immune response, and can include activating an immune response, upregulating an immune response, enhancing an immune response and/or altering an immune response (such as by eliciting a type of immune response which in turn changes the prevalent type of immune response in a subject from one which is harmful or ineffective to one which is beneficial or protective).

The term "operatively linked" refers to linking a nucleic acid molecule to a transcription control sequence in a manner such that the molecule is able to be expressed when transfected (i.e., transformed, transduced or transfected) into a host cell. Transcriptional control sequences are sequences which control the initiation, elongation, and termination of transcription. Particularly important transcription control sequences are those which control transcription initiation, such as promoter, enhancer, operator and repressor sequences. A variety of such transcription control sequences are known to those skilled in the art. Preferred transcription control sequences include those which function in avian, fish, mammalian, bacteria, plant, and insect cells. While any transcriptional control sequences may be used with the invention, the sequences may include naturally occurring transcription control sequences naturally associated with a sequence encoding an immunogen or immune stimulating protein.

The terms "nucleic acid molecule" and "nucleic acid sequence" can be used interchangeably and include DNA, RNA, or derivatives of either DNA or RNA. The terms also include oligonucleotides and larger sequences, including both nucleic acid molecules that encode a protein or a fragment thereof, and nucleic acid molecules that comprise regulatory regions, introns, or other non-coding DNA or RNA. Typically, an oligonucleotide has a nucleic acid sequence from about 1 to about 500 nucleotides, and more typically, is at least about 5 nucleotides in length. The nucleic acid molecule can be derived from any source, including mammalian, fish, bacterial, insect, viral, plant, or synthetic sources. A nucleic acid molecule can be produced by methods commonly known in the art such as recombinant DNA technology (e.g., polymerase chain reaction (PCR), amplification, cloning) or chemical synthesis. Nucleic acid molecules include natural nucleic acid molecules and homologues thereof, including, but not limited to, natural allelic variants and modified nucleic acid molecules in which nucleotides have been inserted, deleted, substituted, or inverted in such a manner that such modifications do not substantially interfere with the nucleic acid molecule's ability to encode an immunogen or immune stimulating protein useful in the methods of the present disclosure. A nucleic acid homologue may be produced using a number of methods known to those skilled in the art (see, for example, Sambrook et al., Molecular Cloning: A Laboratory Manual, Cold Spring Harbor Labs Press, 1989). Techniques to screen for immunogenicity, such as pathogen antigen immunogenicity or cytokine activity are known to those of skill in the art and include a variety of in vitro and in vivo assays.

### EXAMPLES

The following examples illustrate various embodiments of the disclosure.

### Example 1. Materials and Methods.

### Immunomodulator

The immunomodulator used in this study was a composition comprising a cationic lipid and non-coding DNA. The synthetic immunomodulator lipid components [1-[2-[9-(Z)-octadecenoyloxy]]-2-[8](Z)-heptadecenyl]-3-[hydroxyethyl]imidazolinium chloride (DOTIM) and a synthetic neutral lipid cholesterol were formulated to produce liposomes approximately 200 nm in diameter (See, U.S. Patent 6,693,086). The DNA component was a 4292 base-pair non-coding DNA plasmid (pMB 75.6) produced in E. coli, which, being negatively charged, associates with the positively-charged (cationic) liposomes (See, U.S. Patent 6,693,086).

**Table 1. Immunomodulator dilution scheme for doses administered to 600 eggs/group.**

| **Group** | **Target Dose (microgram/egg)** | **Calculated Dose (microgram)** | **Stock Volume (mL)** | **Diluent (D5W) (mL)** | **Total Volume (mL)** | **Dose Volume Per Animal (mL)** |
|---|---|---|---|---|---|---|
| T1 | 0 | 0 | 0 | 25.00 | 25.00 | 0.05 |
| T2 | 0.1 | 0.11 | 0.15 | 24.85 | 25.00 | 0.05 |
| T3 | 1.0 | 1.02 | 1.50 | 23.50 | 25.00 | 0.05 |
| T4 | 10.0 | 9.97 | 14.70 | 10.3 | 25.00 | 0.05 |
| T5 | 0 | 0 | 0 | 25.00 | 25.00 | 0.05 |
| T6 | 1.0 | 1.02 | 1.50 | 23.50 | 25.00 | 0.05 |

### Study Animals

Commercially available chicken eggs (Meat Broilers) were candled to determine viability at 18 days of incubation. Healthy embryos were included in the studies and infertile, dead, and unhealthy embryos were discarded. The chicks were housed in a conventional California style curtain-sided poultry house. Each pen had 50 straight run birds placed on Day 0. The chicks were fed rations that complied with MRC recommendations for the age and weight of the study animals and the chicks had *ad libitum* access to water provided through a bell waterer attached to the water supply for each pen.

### Experimental Infection and Challenge

The chicks were challenged with organisms to determine the efficacy of immune response. The challenge, or experimental infection, included exposure to an inoculum of organisms such as *Escherichia coli* (E.coli). The organisms were used at a concentration of 2.63X10⁵ and administered by spraying 0.15 mL of the inoculums onto each embryonated egg while in the hatching tray.

### Example 2. Administration of the immunomodulator increases hatchability.

A study was conducted to determine the efficacy of an immunomodulator, as described in example 1, administered to 18 day-old embryonated chicken eggs followed by exposure to Escherichia coli (E. coli). The study included two groups (Table 2), one group challenged with E. coli (T1-4) and the other not challenged (T5 and T6). Within the E. coli challenged group, there were four sub-groups that were each administered a different dose of immunomodulator including doses of none (T1), 0.1 µg (T2), 1.0 µg (T3), and 10.0 µg (T4). The non-challenged group included two sub-groups which were either not administered immunomodulator (T5) or were administered 1.0 µg of immunomodulator (T6).

**Table 2. Study Treatment Groups.**

| **Group** | **Immunomodulator** | **E. coli Challenge** |
|---|---|---|
| T1 | 0 | Yes |
| T2 | 0.1 µg/egg | Yes |
| T3 | 1.0 µg/egg | Yes |
| T4 | 10.0 µg/egg | Yes |
| T5 | 0 | No |
| T6 | 1.0 µg/egg | No |

On day 0 of the study, 18 day-old embryonated eggs receiving the immunomodulator were injected in ovo. On day 1, 19 day-old embryonated eggs in the challenged groups (T1-T4) were each sprayed with 0.15 mL of inoculums containing E. coli at a concentration of 2.63X10⁵.

Analysis of all groups resulted in a statistically significant treatment effect on hatchability of the embryonated eggs. There were no significant differences between the two non-challenged control groups (T5, 0 µg/egg at 91% versus T6, 1.0 µg/egg at 89%). In addition, comparison between the two non-treated groups (T1-challenged and T5-nonchallenged) resulted in more embryonated eggs hatching in the non-challenged group (85% versus 91%, respectively). Thus, the challenge model was confirmed as being an effective model for assessing hatching rates.

For those groups treated with the immunomodulator, the T3 (1.0 µg/egg-treated eggs) group hatched more birds (91.5%) than either the non-treated/challenged T1 (84.5%) or the T4 (10.0 µg/egg-treated eggs) (85%) (FIG. 1). Similar differences were shown between the non-treated/non-challenged control group (T5) and these same two treatment groups (T1, as stated above, and T4). No other significant findings were shown between any of the remaining pair-wise group comparisons (FIG. 1).

There was no significant difference on the proportion of birds dying per pen, per group, per study day. (FIG. 2). The proportion of birds surviving on any particular study day resulted in no statistically significant interaction between treatment and time, but did show a significant treatment effect (FIG. 3). Birds in groups T3, T5, and T6 had significantly more birds on any day when compared to groups T1, T2, and T4. In addition, T5 birds also had significantly more alive birds than T3. Assessing the viability of birds after hatch resulted in similar significant findings as above (FIGs. 4-12).

There were no significant differences in pen weights observed on Day 7, 14, 21 or 28 (FIGs. 13-16). Analyses of total pen weights on day 35 resulted in a significant difference between T5 and T2 groups (an average of 87 kgs versus 75 kgs, respectively) (FIG. 17). Pen weights at the end of the study (day 45) were greater for groups T5 (120 kgs) and T6 (117 kgs) when compared to T1 (105 kgs), and T5 when compared to T2 (106 kgs) and T4 (105 kgs) (FIG. 18).

In summary, the effect of the immunomodulator when administered in ovo prior to E. coli infection in commercial broilers was assessed. For those groups treated with the immunomodulator, the T3 group hatched more birds (92%) than either the non-treated/challenged T1 (85%) or the T4 (85%) group. Similar differences were shown between the non-treated/non-challenged control group (T5) and the same treatment groups T1 and T4. There were no significant differences in mortality (FIGs. 2-12) and body weights (FIGs. 13-18). In this study, the immunomodulator was efficacious in increasing the hatchability of commercial broiler eggs challenged with E. coli prior to hatching, especially at the dose of 1.0 µg indicating an enhanced immunity among immunomodulator receiving groups.

### Example 3. Administration of the immunomodulator increases non-antigen-specific immunity.

A study was conducted to determine the effect of an immunomodulator, as described in example 1, in Marek's disease vaccinated chickens exposed to E. coli. The study included two groups, one group challenged with E. coli (T1-T4) and the other not challenged (T5 and T6). Within the E.coli challenged group, there were four sub-groups that were each administered a different dose of immunomodulator including doses of none (T1), 0.1 µg (T2), 1.0 µg (T3), 1.0 µg plus 1 dose of Marek's vaccine (T4) (Table 3). The non-challenged group included two sub-groups which were either not administered immunomodulator (T5) or were administered 1.0 µg of immunomodulator (T6).

**Table 3. Study treatment groups.**

| **Group** | **In ovo Administration** | **Number of embryonated eggs** | **Number of pens** |
|---|---|---|---|
| T1 | 0 µg Immunomodulator/egg | 1000 | 12 |
| T2 | 0.1 µg Immunomodulator/egg | 1000 | 12 |
| T3 | 1.0 µg Immunomodulator/egg | 1000 | 12 |
| T4 | 1.0 µg Immunomodulator + 1 dose of Marek's Vaccine/egg | 1000 | 12 |
| T5 | 0 µg Immunomodulator/egg | 1000 | 12 |
| T6 | 1.0 µg Immunomodulator/egg | 1000 | 12 |

Commercial broiler eggs were incubated for 18 days, candled for viability and then 18 day-old embryonated eggs receiving the immunomodulator were injected in ovo. Next, 19 day-old embryonated eggs in the challenged groups were each sprayed with 0.15 mL of inoculum containing E. coli at a concentration of 2.8X10⁷ per milliliter. The eggs hatched on day 0 of the study, which extended for 45 days.

The number of embyronated eggs hatched were recorded for each group (n=1000 per group or hatching tray) (FIG. 19). Of the chicks hatched, the number of alive chicks remaining on day 7 after hatch was recorded (FIG. 20). The number of live/dead chicks per group on day 7 after hatch was also recorded (FIG. 21). The mortality percentage decreased with increasing immunomodulator in the challenged groups and the co-administration of the Marek's vaccine with the immunomodulator further decreased the mortality percentage (FIG. 22).

The data demonstrate that there was no reduction in hatchability between immunomodulator treated groups and non-treated groups that were not challenged with E. coli (FIG. 23). Hatchability was significantly higher between immunomodulator treated groups and non-treated groups that were challenged with E. coli. The hatchability of embryonated eggs receiving the immunomodulator and a dose of Marek's vaccine was similar to that of the non-treated and non-challenged control. In summary, the immunomodulator increases hatchability under challenge conditions and the immunomodulator's protective response is enhanced with the co-administration of Marek's vaccine. The results indicate that a non-antigen-specific immune response is elicited with the administration of the immunomodulator or the immunomodulator and Marek's vaccine.

### Example 4. Administration of the immunomodulator elicits a non-antigen-specific immune response that is enhanced with additional administration of a biological agent.

A study was conducted to determine the effect of an immunomodulator in Marek's disease vaccinated chickens exposed to E. coli. The study included nine groups which were differentially treated as indicated in Table 4.

**Table 4. Study Group Treatments**

| **Group** | **In ovo Administration** | **Number Eggs** | **Marek's disease vaccination (18 day old embryos)** | **18 day old embryos treated with immunomodulator** | **19 day old embryos challenged with E. coli** | **Day 0 chicken treated with immunomodulator** |
|---|---|---|---|---|---|---|
| T1 | 0 µg Immunomodulator/ egg | 400 | Yes | No | No | No |
| T2 | 0 µg Immunomodulator/ egg | 400 | Yes | No | Yes | No |
| T3 | 1.0 µg Immuno-modulatory egg | 400 | Yes | No | No | Yes |
| T4 | 1.0 µg Immuno-modulatory egg | 400 | Yes | No | Yes | Yes |
| T5 | 1.0 µg Immuno-modulatory egg | 400 | Yes | Yes | No | No |
| T6 | 1.0 µg Immuno-modulatory egg | 400 | Yes | Yes | Yes | No |
| T7 | 1.0 µg Immuno-modulatory egg | 400 | Yes | Yes | No | Yes |
| T8 | 1.0 µg Immuno-modulatory egg | 400 | Yes | Yes | Yes | Yes |
| T9 | 0 µg Immuno-modulatory egg | 400 | No | No | No | No |

Commercial broiler eggs were incubated for 18 days, candled for viability and then 18 day-old embryonated eggs receiving the immunomodulator were injected in ovo. Next, 19 day-old embryonated eggs in the challenged groups were each sprayed with 0.15 mL of inoculums containing E. coli at a concentration of 2.63 x 10⁵ per milliliter. The eggs hatched on day 0 of the study, which extended for 45 days.

The percentage of embyronated eggs hatched was calculated for each group (FIG. 24). Of the chicks hatched, the mortality percentage was calculated on day 7 after hatch for both challenged (FIG. 26) and non-challenged (FIG. 25) groups. The challenged groups demonstrated higher mortality percentages than similarly treated non-challenged groups (FIG. 27). The percentage of live chicks per group was calculated from hatch to day 7 (FIG. 28) as well as day 3 (18 day live embryo) to day 7 (FIG. 29, n=400). There was a significantly higher survival rate for birds treated with immunomodulator prior to challenge (>98.2%, FIG. 28) compared to the control group that was challenged (82.2%, FIG. 28). Also, there was a significantly higher survival rate on day 7 for birds treated at hatch with immunomodulator before and after challenge at day 7 (95%, FIG. 28) compared to challenged control groups (82.2%, FIG. 28). These results were recapitulated using a set containing 400 eggs per group (FIG. 29). There was a higher survival rate on day 7 for treated embryos prior to challenge (set 400) (>94.3%, Fig. 29) compared to control group (67.0%, Fig. 29). Similarly, there was a significantly higher survival rate for birds treated with immunomodulator in ovo (97.4%, FIG. 30, no second treatment, at day 14 compared to challenged control group (81.9%, FIG. 30). There was significantly higher live birds at day 14 in the group treated with the immunomodulator on day 1 of age, post-challenge, 93.9% (FIG. 30), no first treatment, compared to control group 81.9% (FIG. 30). There was also significantly higher live birds at day 14 in the group treated with the immunomodulator in ovo and then on day 1 of age, 95.8% (FIG. 30), compared to the control group, 81.9% (FIG.30). Fig. 30 shows the weekly mortality of the challenged and not-challenged groups with different treatments.

The data demonstrate that there was no reduction in hatchability between immunomodulator treated groups (treated once or twice) and non-treated groups that were not challenged with E. coli. Hatchability was higher in groups that were treated with the immunomodulator prior to challenge with E. coli. This effect was enhanced when the immunomodulator was co-administered with Marek's vaccine. Also, immunomodulator treatment correlated to a decrease in mortality among non-challenged groups. Mortality was further decreased with co-administration of Marek's vaccine and pretreatment with the immunomodulator prior to administration of the immunomodulator with Marek's vaccine. These results were recapitulated in the challenged groups. In summary, the immunomodulator increases hatchability under challenge conditions and the immunomodulator's protective response is enhanced with the co-administration of Marek's vaccine. The results indicate that the non-antigen-specific immune response elicited by administration of the immunomodulator is enhanced when its administration is accompanied by the Marek's vaccine. Co-administration of the immunomodulator with Marek's vaccine further enhances the non-antigen-specific immune response.

### Example 5. Administration of the immunomodulator after infection that elicits a non-antigen-specific immune response that is enhanced with additional administration of a biological agent.

There was a significantly higher survival rate for birds treated with immunomodulator after challenge (95%, FIG. 28) compared to challenged control groups (82.2%, FIG. 28). These results were recapitulated using a set containing 400 eggs per group (FIG. 29).

The results indicate that the non-antigen-specific immune response elicited by administration of the immunomodulator after E. coli challenge is enhanced when its administration is accompanied by the Marek's vaccine. Post-administration of the immunomodulator after the Marek's vaccine enhanced the non-antigen-specific immune response.

### Example 6. Administration of the immunomodulator with a bivalent biological agent does not inhibit early replication of the bivalent biological agent

A study was conducted to determine if an immunomodulator would negatively impact early replication of Marek's disease bivalent vaccine. Marek's disease bivalent vaccine includes HVT (turkey herpes virus) and SB1 (chicken herpes virus).

Commercial broiler eggs were incubated for 18 days, candled for viability. The eggs were separated into 3 groups, A-C with 20 eggs per group. Each egg was vaccinated at indicated in Table 5.

**Table 5: Treatment**

| Treatment Group | In ovo Administration | Number of 18 day old embryonated eggs (eggs that hatched) | Marek's disease vaccination (18 day old embryos) | 18 day old embryos treatment with Immunomudulator |
|---|---|---|---|---|
| A | 0 µg immunomodulator /egg | 20 (12) | HVT-SB1 | No |
| B | 1.0 µg immunomodulator /egg | 20 (18) | HVT-SB1 | Yes |
| C | 0 µg immunomodulator /egg | 20 (14) | None | No |

To assess the effect of the immunomodulator on vaccine virus replication in vivo, virus reisolation was performed at 7 and 14 days post placement from both spleen cells (SPC) and peripheral blood mononuclear cells (PBMC).

As MD vaccine virus infection is known for bird-to-bird variation in the level of virus replication, reisolation was performed using triplicate pools of chickens (2 chickens per pool, 3 pools per sampling point).

Despite the variability among pools, there is a clear trend in the replication of HVT and SB-1 in the presence of the immunomodulator. This data was most significant for SB-1 replication during the first week, but the overall trends for HVT and SB-1 were similar for both tissues at both time points (7 and 14 days post hatch). The data is summarized in Fig. 31-38. Fig. 31 shows the effect of the immunomodulator on MD vaccine replication of HVT in Spleen Cells in Week 1. Fig. 32 shows the effect of the immunomodulator on MD vaccine replication of HVT in PBMC in Week 1. Fig. 33 shows the effect of the immunomodulator on MD vaccine replication of SB-1 in Spleen Cells in Week 1. Fig. 34 shows the effect of the immunomodulator on MD vaccine replication of SB-1 in PBMC in Week 1. Fig. 35 shows the effect of the immunomodulator on MD vaccine replication of HVT in Spleen Cells in Week 2. Fig. 36 shows the effect of the immunomodulator on MD vaccine replication of HVT in PBMC in Week 2. Fig. 37 shows the effect of the immunomodulator on MD vaccine replication of SB-1 in Spleen Cells in Week 2. Fig. 38 shows the effect of the immunomodulator on MD vaccine replication of SB-1 in PBMC in Week 2.

The immunomodulator does not have a negative impact on MD vaccine replication. The immunomodulator appears to have an adjuvant effect that increases the replication of HVT and SB-1 at the first two weeks post-infection. This data suggests that the immunomodulator would have a positive impact on MD vaccine efficacy.

### Example 7. Administration of the immunomodulator with a modified live biological agent does interfere with of the modified live biological agent

A study was conducted to determine the effect of an immunomodulator with a modified live New Castle's disease vaccinated chickens exposed to New Castle Disease. The study included nine groups which were differentially treated as indicated in Table 6.

**Table 6. Study Group Treatments**

| Day | Treatment | T1 | T2 | T3 | T4 | T5 | T6 | T7 | T8 | T9 |
|---|---|---|---|---|---|---|---|---|---|---|
| -3 | Immunomodulator (µg) in ovo | | | | 0.112 | 0.56 | 1.12 | | | |
| | PBS in ovo | X | X | X | | | | X | X | X |
| 0 | Randomly assign chicks to be included in study | X | X | X | X | X | X | X | X | X |
| | Obtain blood for anti-NDV titers from chicks culled from study | X | X | X | X | X | X | X | X | X |
| | Immunomodulator (µg) by aerosol | | | | | | | 0.112 | 0.56 | 1.12 |
| | Immunomodulator aerosol | | | X | X | X | X | X | X | X |
| | PBS aerosol | X | X | | | | | | | |
| 7 | Obtain blood for anti-NDV titers | X | X | X | X | X | X | X | X | X |
| 14 | Obtain blood for anti-NDV titers | X | X | X | X | X | X | X | X | X |
| 21 | Obtain blood for anti-NDV titers | X | X | X | X | X | X | X | X | X |
| | Challenge with NDV | | X | X | X | X | X | X | X | X |
| 22-26 | Observe for clinical signs of NDV infection | X | X | X | X | X | X | X | X | X |
| 26 | Gross pathology for air sacculitis | X | X | X | X | X | X | X | X | X |
| | Tracheal histopathology | X | X | X | X | X | X | X | X | X |

Commercial broiler eggs were incubated for 18 days, candled for viability. The eggs were separated into 9 groups, T1-T9 with 60 eggs per group. Group T9 suffered a vermin attack the first night after hatching and there were some deaths from unknown causes over the course of the study. At the time of culling, the number of birds per group was as listed on Table 7.

**Table 7. Birds per group**

| Group | Treatment | Number of Birds |
|---|---|---|
| 1 | No treatment, No challenge | 60 |
| 2 | Challenge Only | 60 |
| 3 | Vaccine + Challenge | 60 |
| 4 | Vaccine + Challenge + 0.112 µg Immunomodulator in ovo | 58 |
| 5 | Vaccine + Challenge + 0.56 µg Immunomodulator in ovo | 58 |
| 6 | Vaccine + Challenge + 1.12 µg Immunomodulator in ovo | 57 |
| 7 | Vaccine + Challenge + 0.112 µg Immunomodulator aerosol | 60 |
| 8 | Vaccine + Challenge + 0.56 µg Immunomodulator aerosol | 59 |
| 9 | Vaccine + Challenge + 1.12 µg Immunomodulator aerosol | 50 |

The 18 day-old embryonated eggs in groups T4-T6 were injected with the immunomodulator in ovo. Groups T1-T3 and T7-T9 were injected with saline. On day 0, the eggs hatched and the chicks in groups T1-T2 were sprayed with saline, groups T3-T6 received the New Castle Vaccine, Newhatch-C2 manufactured by Intervet/Schering-Plough Animal Health, groups T7-T9 were sprayed with immunomodulator and vaccinated.

Serum was obtained for anti-NDV titers at days 7, 14, and 21 after hatch. At day 21 the chicks Groups T2-T9 were challenged with lentogenic Newcastle disease virus. At day 26 the chicks were culled, bled, examination for gross pathology for air sacculitis, and the serum analysis was conducted.

The data demonstrate that there was no significant difference in serology between the groups on days 7, 14, and 21. However, on day 26, which was 5 days post challenge, birds that received the immunomodulator in ovo had dramatically increased anti-NDV titers over all other groups including vaccine alone (Fig 40).

Air sacculitis was dramatically reduced between T2-T5, the chicks that received the in ovo modulator (Fig 39). The results indicate that in-ovo administration of the immunomodulator does not interfere with NDV vaccination.

### Example 8. Effectiveness of administration of the immunomodulator with biological agent in a virulent challenge

A study was conducted to evaluate the interference of a single dose of immunomodulator with routine Marek's disease vaccination in 18 day old embryonated eggs. The study compared the efficacy of Marek's disease vaccination against a virulent challenge.

A total of 160 embryonated eggs were split into 8 groups. At hatch each shedder bird was inoculated with Marek's disease virus, day 0 of the study. The number of shedder birds that hatched was 148 out of 160 (see table 8 below for starting number of eggs/birds versus actual birds at the end of the study in parantheses). After three weeks, 80 more birds were added to each group, 15 unvaccinated and untreated birds (contacts) and 65 treated birds (vaccinates) (see table 8 below for treatment of each group).

**Table 8: Treatment**

| Room | Group | Vaccine | Dose | Route | Shedder | Contacts | Vaccinates |
|---|---|---|---|---|---|---|---|
| 2 | A | HVT/SB1 | 1X | in ovo | 20 (19) | 15 (15) | 65 (59) |
| 3 | B | HVT/SB1 + Immunomodulator | 1X | in ovo | 20 (19) | 15 (15) | 65 (58) |
| 4 | A | HVT/SB1 | 1X | in ovo | 20 (19) | 15 (15) | 65 (58) |
| 5 | B | HVT/SB1 + Immunomodulator | 1X | in ovo | 20 (19) | 15 (15) | 65 (58) |
| 6 | A | HVT/SB1 | 1X | in ovo | 20 (18) | 15 (14) | 65 (59) |
| 7 | B | HVT/SB1 + Immunomodulator | 1X | in ovo | 20 (18) | 15 (14) | 65 (58) |
| 8 | A | HVT/SB1 | 1X | in ovo | 20 (18) | 15 (14) | 65 (59) |
| 9 | B | HVT/SB1 + Immunomodulator | 1X | in ovo | 20 (18) | 15 (14) | 65 (57) |

At day 42 of the study, the surviving shedder birds were removed and necropsied. At day 63 of the study (day 42 for the contacts and vaccinates) the surviving study birds were removed and neocropsied.

Figure 41 shows the survival curves of inoculated Shedder birds, Unvaccinated Contact birds, vaccinated birds only, vaccinated/immunomodulator birds. The unvaccinated contact data is the mean of 8 pens, while each vaccinated group is the mean of 4 pens each. As can be observed, there is a similarity in the slope of the survival curves for the vaccinated only and vaccinated/immunomodulator birds.

Figure 42 shows the mean incidence of Marek's Disease among the inoculated Shedder birds, Unvaccinated Contact birds, vaccinated birds only, vaccinated/immunomodulator birds. Both the shedders and contact data are the mean of 8 pens, while vaccinated groups are the mean of 4 pens each. As can be observed, the vaccinated only group and the vaccinated/immunomodulator group had a much lower incidence of Marek's Disease. The vaccinated/ immunomodulator group had a lower incidence of Marek's Disease than the vaccinated only group.

As can be seen from Figures 41-42 the immunomodulator does not have a deleterious effect on the Marek's Disease vaccine protection. In fact, it elicited an increase in vaccine efficacy.

## Claims

1. An immunomodulator composition, wherein the immunomodulator composition comprises:
a. a cationic liposome delivery vehicle; and
b. a DNA plasmid that does not code for an immunogen,
for use in preventing a reduction in survival rate when co-administered with a vaccine of a chicken hatched from an embryonated chicken egg, by administration *in ovo* to the egg of an effective amount of 0.05 to 10 micrograms, preferably 0.1 to 5 micrograms, of the immunomodulator composition,
wherein the vaccine is a vaccine used for protection against Marek's disease virus (MDV).

2. The composition for use of claim 1, wherein the DNA plasmid is an isolated bacterially-derived nucleic acid vector without a gene insert, or a fragment thereof.

3. The composition for use of any of claims 1 to 2, wherein the immunomodulator composition is administered to an embryonated chicken egg at day 18 of incubation.

4. The composition for use of any of claims 1 to 3, wherein the liposome delivery vehicle comprises lipids selected from the group consisting of multilamellar vesicle lipids and extruded lipids.

5. The composition for use of any of claims 1 to 4, wherein the cationic liposome delivery vehicle comprises pairs of lipids selected from the group consisting of DOTMA and cholesterol; DOTAP and cholesterol; DOTIM and cholesterol; and DDAB and cholesterol.

6. The composition for use of any of claims 1 to 5, wherein the cationic liposome delivery vehicle comprises DOTIM and cholesterol.

## Patentansprüche

1. Eine Immunmodulator-Zusammensetzung, wobei die Immunmodulator-Zusammensetzung umfasst:
a. eine kationische Liposomabgabe-Trägersubstanz; und
b. ein DNS Plasmid, das nicht für ein Immunogen kodiert,
zur Verwendung in der Prävention einer Reduktion in der Überlebensrate in einem Huhn, das aus einem embryonierten Hühnerei geschlüpft ist, wenn mit einem Impfstoff zusammen verabreicht, mittels *in ovo* Verabreichung an das Ei in einer wirksamen Menge von 0,05 bis 10 Mikrogramm, vorzugsweise 0,1 bis 5 Mikrogramm an Immunmodulator-Zusammensetzung,
wobei der Impfstoff ein Impfstoff ist, der zum Schutz gegen das Virus der Marek-Krankheit (MDV) verwendet wird.

2. Die Zusammensetzung zur Verwendung gemäß Anspruch 1, wobei das DNS Plasmid ein isolierter, bakteriell abgeleiteter Nukleinsäurevektor ohne einen Gen-Insert oder ein Fragment davon ist.

3. Die Zusammensetzung zur Verwendung gemäß einem der Ansprüche 1 bis 2, wobei die Immunmodulator-Zusammensetzung einem embryonierten Hühnerei an Tag 18 der Inkubation verabreicht wird.

4. Die Zusammensetzung zur Verwendung gemäß einem der Ansprüche 1 bis 3, wobei die Liposomabgabe-Trägersubstanz Lipide ausgewählt ist aus der Gruppe bestehend aus multilamellaren Vesikellipiden und extrudierten Lipiden umfasst.

5. Die Zusammensetzung zur Verwendung gemäß einem der Ansprüche 1 bis 4, wobei die kationische Liposomabgabe-Trägersubstanz Lipidpaare ausgewählt aus der Gruppe bestehend aus DOTMA und Cholesterin; DOTAP und Cholesterin; DOTIM und Cholesterin; und DDAB und Cholesterin umfasst.

6. Die Zusammensetzung zur Verwendung gemäß einem der Ansprüche 1 bis 5, wobei die kationische Liposomabgabe-Trägersubstanz DOTIM und Cholesterin umfasst.

## Revendications

1. Une composition immunomodulatrice, dans laquelle la composition immunomodulatrice comprend :
a. un véhicule de distribution de liposomes cationique ; et
b. un plasmide d'ADN qui ne code pas pour un immunogène,
destinée à être utilisée pour la prévention d'une réduction du taux de survie lorsqu'elle est co-administrée avec un vaccin d'une poule issue d'un œuf de poule embryonné, par administration *in ovo* à l'œuf d'une quantité efficace de 0,05 à 10 microgrammes, de préférence 0,1 à 5 microgrammes, de la composition immunomodulatrice,
le vaccin étant un vaccin utilisé pour la protection contre le virus de la maladie de Marek (MDV).

2. La composition pour l'utilisation selon la revendication 1, dans laquelle le plasmide d'ADN est un vecteur d'acide nucléique isolé dérivé d'une bactérie sans insert de type gène, ou un fragment de celui-ci.

3. La composition pour l'utilisation selon l'une quelconque des revendications 1 à 2, dans laquelle la composition immunomodulatrice est administrée à un œuf de poule embryonné au 18^{e} jour d'incubation.

4. La composition pour l'utilisation selon l'une quelconque des revendications 1 à 3, dans laquelle le véhicule de distribution de liposomes comprend des lipides choisis dans le groupe constitué par les lipides de vésicules multilamellaires et les lipides extrudés.

5. La composition pour l'utilisation selon l'une quelconque des revendications 1 à 4, dans laquelle le véhicule de distribution de liposomes cationique comprend des paires de lipides choisis dans le groupe constitué par le DOTMA et le cholestérol ; le DOTAP et le cholestérol ; le DOTIM et le cholestérol ; et le DDAB et le cholestérol.

6. La composition pour l'utilisation selon l'une quelconque des revendications 1 à 5, dans laquelle le véhicule de distribution de liposomes cationique comprend du DOTIM et du cholestérol.
